# EUROPEAN PATENT SPECIFICATION

(11) **EP 4 085 048 B1**
(45) Date of publication and mention of the grant of the patent: **13.11.2024**
(21) Application number: 20841940.8
(22) Date of filing: 24.12.2020
(51) Int. Cl.: C07C 255/53, A01N 43/00, C07C 255/54, C07C 255/55, C07C 255/57, C07C 255/59, C07C 317/22, C07C 317/28, C07D 207/00

(54) **HERBICIDAL COMPOUNDS**
HERBIZIDE VERBINDUNGEN
COMPOSÉS HERBICIDES

(30) Priority: 02.01.2020 GB 202000011
(43) Date of publication of application: 09.11.2022
(73) Proprietor: Syngenta Crop Protection AG, 4058 Basel (CH)
(72) Inventor: MORRIS, James, Alan, Bracknell Berkshire RG42 6EY (GB); INGRAM, Katharine, Mary, Bracknell Berkshire RG42 6EY (GB); DALE, Suzanna, Jane, Bracknell Berkshire RG42 6EY (GB); BURNS, David, Bracknell Berkshire RG42 6EY (GB)
(74) Representative: SYNGENTA IP
(86) International application number: PCT/EP2020/087853
(87) International publication number: WO 2021/136742

(56) References cited:
- EP-A1- 0 820 996
- WO-A1-2008/156715
- WO-A1-96/06089
- ABRAMOVITCH R A ET AL, JOURNAL OF ORGANIC CHEMISTRY, AMERICAN CHEMICAL SOCIETY, WASHINGTON, vol. 41, no. 10, 1 January 1976 (1976-01-01), pages 1717 - 1724, XP002081541, ISSN: 0022-3263, DOI: 10.1021/JO00872A011
- HAROLD W. MOORE ET AL: "Chemistry of azido quinones. Cyanophenols from 4-alkynyl-3-azido-1,2-benzoquinones", THE JOURNAL OF ORGANIC CHEMISTRY, vol. 52, no. 12, 1 June 1987 (1987-06-01), pages 2530 - 2537, XP055130635, ISSN: 0022-3263, DOI: 10.1021/jo00388a034

## Description

The present invention relates to novel herbicidal compounds, to processes for their preparation, to herbicidal compositions which comprise the novel compounds, and to their use for controlling weeds, in particular in crops of useful plants, or for inhibiting plant growth.

EP 0 820 996 A1 discloses herbicidal cyanopyridines. WO96/06089 teaches substituted biphenyl derivatives and their use as herbicides. It has now been found that certain intermediates that are described in the synthesis of the biphenyl derivatives therein are themselves herbicidal. Thus, according to the present invention there is provided a compound of Formula (I): 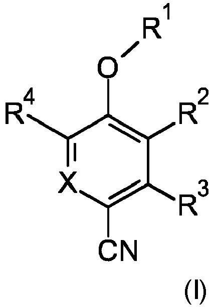 or an agronomically acceptable salt thereof,
wherein
X is CH or N,
R¹ is selected from the group consisting of hydrogen, C₁-C₁₀alkylC(O)-, C₁-C₁₀alkylOC(O)-, C₁-C₁₀alkylSC(O)- and R⁵R⁶NC(O)-;
R² is selected from the group consisting of halogen, NO₂, CN, C₁-C₆haloalkylS(O)ₚ-, C₁-C₆alkyl-S(O)ₚ-, C₂-C₄alkenyl-, C₂-C₄alkynyl-, C₁-C₄haloalkyl and C₁-C₄haloalkoxy-;
R³ is selected from the group consisting of hydrogen, halogen, C₁-C₄alkyl, C₁-C₄haloalkyl, C₁-C₄alkoxy- and C₁-C₄haloalkoxy-;
R⁴ is phenyl or a five or six-membered heteroaryl, the heteroaryl containing from one to three heteroatoms each independently selected from the group consisting of oxygen, nitrogen and sulphur, and wherein the phenyl or heteroaryl may be optionally substituted by one or more substituents independently selected from the group consisting of halogen, C₁-C₆alkyl-, C₂-C₄alkenyl-, C₂-C₄alkynyl-, C₁-C₆haloalkyl-, C₁-C₆alkoxy-, C₁-C₆alkoxyC₁-C₃alkyl-, C₁-C₆alkoxyC₁-C₃alkoxy-, C₁-C₆haloalkoxy-, C₁-C₆alkyl-S(O)ₚ-, C₁-C₆haloalkyl-S(O)ₚ-, C₃-C₆cycloalkyl- (optionally substituted by 1 or 2 halogen), cyano and nitro; or
R⁴ is R4^{a} 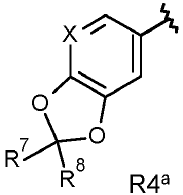 and
R⁵ is hydrogen or C₁-C₁₀alkyl;
R⁶ is hydrogen or C₁-C₁₀alkyl;
R⁷ and R⁸ are independently selected from the group consisting of hydrogen and halogen; and
p = 0, 1 or 2;
wherein said compound is not selected from the group consisting of 2-(3-bromo-5-cyano-2-hydroxyphenyl)pyridine, 3-chloro-2-ethoxy-4-hydroxy-5-phenyl-benzonitrile, 4-hydroxy-3-nitro-5-[3-(trifluoromethyl)phenyl]benzonitrile and 2'-fluoro-6-hydroxy-5-nitrobiphenyl-3-carbonitrile.

C₁-C₄alkyl- includes, for example, methyl (Me, CH₃), ethyl (Et, C₂H₅), n-propyl (*n*-Pr), isopropyl (*i*-Pr), *n*-butyl (*n*-Bu), isobutyl (*i*-Bu), sec-butyl and tert-butyl (*t*-Bu). C₁-C₂alkyl is methyl (Me, CH₃) or ethyl (Et, C₂H₅).

Halogen (or halo) includes, for example, fluorine, chlorine, bromine or iodine. The same correspondingly applies to halogen in the context of other definitions, such as haloalkyl.

C₁-C₆haloalkyl- includes, for example, fluoromethyl, difluoromethyl, trifluoromethyl, chloromethyl, dichloromethyl, trichloromethyl, 2,2,2-trifluoroethyl, 2-fluoroethyl, 2-chloroethyl, pentafluoroethyl, 1,1-difluoro-2,2,2-trichloroethyl, 2,2,3,3-tetrafluoropropyl and 2,2,2-trichloroethyl, heptafluoro-n-propyl and perfluoro-n-hexyl. C₁-C₄haloalkyl- and C₁-C₂haloalkyl include, for example, fluoromethyl, difluoromethyl, trifluoromethyl, chloromethyl, dichloromethyl, trichloromethyl, 2,2,2-trifluoroethyl, 2-fluoroethyl, 2-chloroethyl, pentafluoroethyl, or 1,1-difluoro-2,2,2-trichloroethyl.

C₁-C₄alkoxy and C₁-C₂alkoxy includes, for example, methoxy and ethoxy.

C₁-C₆haloalkoxy- and C₁-C₄haloalkoxy- include, for example, fluoromethoxy, difluoromethoxy, trifluoromethoxy, 2,2,2-trifluoroethoxy, 1,1,2,2-tetrafluoroethoxy, 2-fluoroethoxy, 2-chloroethoxy, 2,2-difluoroethoxy or 2,2,2-trichloroethoxy, preferably difluoromethoxy, 2-chloroethoxy or trifluoromethoxy.

C₂-C₄alkenyl- includes, for example, -CH=CH₂ (vinyl) and -CH₂-CH=CH₂ (allyl).

C₂-C₄alkynyl- refers to a straight or branched hydrocarbon chain radical group consisting solely of carbon and hydrogen atoms, containing at least one triple bond, having from two to four carbon atoms, and which is attached to the rest of the molecule by a single bond. Examples of C₂₋C₄alkynyl include, but are not limited to, prop-1-ynyl, propargyl (prop-2-ynyl), and but-1-ynyl.

C₁-C₄alkyl-S- (alkylthio) includes, for example, methylthio, ethylthio, propylthio, isopropylthio, n-butylthio, isobutylthio, sec-butylthio or tert-butylthio, preferably methylthio or ethylthio.

C₁-C₄alkyl-S(O)- (alkylsulfinyl) includes, for example, methylsulfinyl, ethylsulfinyl, propylsulfinyl, isopropylsulfinyl, n-butylsulfinyl, isobutylsulfinyl, sec-butylsulfinyl or tert-butylsulfinyl, preferably methylsulfinyl or ethylsulfinyl.

C₁-C₄alkyl-S(O)₂- (alkylsulfonyl) includes, for example, methylsulfonyl, ethylsulfonyl, propylsulfonyl, isopropylsulfonyl, n-butylsulfonyl, isobutylsulfonyl, sec-butylsulfonyl or tert-butylsulfonyl, preferably methylsulfonyl or ethylsulfonyl.

C₃-C₆alkyl- includes, for example, cyclopropyl and cyclohexyl.

Five or six-membered heteroaryl includes, for example, furanyl, thiophenyl, thiazolyl, oxazolyl, isoxazolyl, thiazolyl, pyrazolyl, isothiazolyl, oxadiazolyl, pyridyl, pyridazinyl, pyrazinyl, pyrimidinyl, thiadiazolyl and triazolyl.

In one embodiment of the present invention there is provided a compound of Formula (I) wherein R¹ is C₁-C₆alkylC(O)- (e.g CH₃C(O)-, iPrC(O)-, t-butylC(O)-).

In another embodiment of the present invention there is provided a compound of Formula (I) wherein R¹ is hydrogen.

In another embodiment of the present invention there is provided a compound of Formula (I) wherein R² is selected from the group consisting of C₁-C₄haloalkyl, halogen and NO₂, more preferably C₁-C₄haloalkyl (e.g C₁-C₄fluoroalkyl such as CF₃, CF₂H). In another preferred embodiment, R² is selected from the group consisting of Br, Cl, CF₂H, -CF₃ and -OCF₃, most preferably CF₃.

In another embodiment of the present invention there is provided a compound of Formula (I) wherein R³ is selected from the group consisting of hydrogen, fluoro, chloro and methyl, most preferably hydrogen.

In another embodiment of the present invention there is provided a compound of Formula (I) wherein R⁴ is phenyl which is optionally substituted as outlined herein.

In another embodiment of the present invention there is provided a compound of Formula (I) wherein R⁴ a five or six-membered heteroaryl selected from the group consisting of furanyl, thiophenyl, thiazolyl, oxazolyl, isoxazolyl, thiazolyl, pyrazolyl, isothiazolyl, pyridyl, pyridazinyl, pyrazinyl, pyrimidinyl and triazolyl which is optionally substituted as defined herein. In a more preferred embodiment R⁴ is selected from the group consisting of phenyl, pyrazolyl (C or N linked), thiazolyl and thiophenyl which is optionally substituted as defined herein.

Where R⁴ is phenyl or a five or six-membered heteroaryl said phenyl or heteroaryl may be optionally substituted as described. For example, the phenyl or heteroaryl may comprise one, two, three or four substituents (depending on the nature of the heteroaryl). Where present, each substituent is preferably independently selected from the group consisting of halogen (e.g F, CI), C₁-C₆haloalkyl- (e.g CF₃, CF₂H), C₁-C₆alkyl- (e.g methyl, ethyl), C₃-C₆cycloalkyl- (e.g cyclopropyl), cyano and C₁-C₆alkoxy- (e.g methoxy-, ethoxy-).

In another embodiment of the present invention R⁷ and R⁸ are independently selected from hydrogen and fluorine.

In another embodiment of the present invention there is provided a compound of Formula (I) wherein X is N.

In another embodiment of the present invention there is provided a compound of Formula (I) wherein X is CH.

Compounds of Formula (I) may contain asymmetric centres and may be present as a single enantiomer, pairs of enantiomers in any proportion or, where more than one asymmetric centre are present, contain diastereoisomers in all possible ratios. Typically one of the enantiomers has enhanced biological activity compared to the other possibilities.

The present invention also provides agronomically acceptable salts of compounds of Formula (I). Salts that the compounds of Formula (I) may form with amines, including primary, secondary and tertiary amines (for example ammonia, dimethylamine and triethylamine), alkali metal and alkaline earth metal bases, transition metals or quaternary ammonium bases are preferred.

The compounds of Formula (I) according to the invention can be used as herbicides by themselves, but they are generally formulated into herbicidal compositions using formulation adjuvants, such as carriers, solvents and surface-active agents (SAA). Thus, the present invention further provides a herbicidal composition comprising a herbicidal compound according to any one of the previous claims and an agriculturally acceptable formulation adjuvant. The composition can be in the form of concentrates which are diluted prior to use, although ready-to-use compositions can also be made. The final dilution is usually made with water, but can be made instead of, or in addition to, water, with, for example, liquid fertilisers, micronutrients, biological organisms, oil or solvents.

The herbicidal compositions generally comprise from 0.1 to 99 % by weight, especially from 0.1 to 95 % by weight, compounds of Formula I and from 1 to 99.9 % by weight of a formulation adjuvant which preferably includes from 0 to 25 % by weight of a surface-active substance.

The compositions can be chosen from a number of formulation types. These include an emulsion concentrate (EC), a suspension concentrate (SC), a suspoemulsion (SE), a capsule suspension (CS), a water dispersible granule (WG), an emulsifiable granule (EG), an emulsion, water in oil (EO), an emulsion, oil in water (EW), a micro-emulsion (ME), an oil dispersion (OD), an oil miscible flowable (OF), an oil miscible liquid (OL), a soluble concentrate (SL), an ultra-low volume suspension (SU), an ultra-low volume liquid (UL), a technical concentrate (TK), a dispersible concentrate (DC), a soluble powder (SP), a wettable powder (WP) and a soluble granule (SG). The formulation type chosen in any instance will depend upon the particular purpose envisaged and the physical, chemical and biological properties of the compound of Formula (I).

Soluble powders (SP) may be prepared by mixing a compound of Formula (I) with one or more water-soluble inorganic salts (such as sodium bicarbonate, sodium carbonate or magnesium sulphate) or one or more water-soluble organic solids (such as a polysaccharide) and, optionally, one or more wetting agents, one or more dispersing agents or a mixture of said agents to improve water dispersibility/solubility. The mixture is then ground to a fine powder. Similar compositions may also be granulated to form water soluble granules (SG).

Wettable powders (WP) may be prepared by mixing a compound of Formula (I) with one or more solid diluents or carriers, one or more wetting agents and, preferably, one or more dispersing agents and, optionally, one or more suspending agents to facilitate the dispersion in liquids. The mixture is then ground to a fine powder. Similar compositions may also be granulated to form water dispersible granules (WG).

Granules (GR) may be formed either by granulating a mixture of a compound of Formula (I) and one or more powdered solid diluents or carriers, or from preformed blank granules by absorbing a compound of Formula (I) (or a solution thereof, in a suitable agent) in a porous granular material (such as pumice, attapulgite clays, fuller's earth, kieselguhr, diatomaceous earths or ground corn cobs) or by adsorbing a compound of Formula (I) (or a solution thereof, in a suitable agent) on to a hard core material (such as sands, silicates, mineral carbonates, sulphates or phosphates) and drying if necessary. Agents which are commonly used to aid absorption or adsorption include solvents (such as aliphatic and aromatic petroleum solvents, alcohols, ethers, ketones and esters) and sticking agents (such as polyvinyl acetates, polyvinyl alcohols, dextrins, sugars and vegetable oils). One or more other additives may also be included in granules (for example an emulsifying agent, wetting agent or dispersing agent).

Dispersible Concentrates (DC) may be prepared by dissolving a compound of Formula (I) in water or an organic solvent, such as a ketone, alcohol or glycol ether. These solutions may contain a surface active agent (for example to improve water dilution or prevent crystallisation in a spray tank).

Emulsifiable concentrates (EC) or oil-in-water emulsions (EW) may be prepared by dissolving a compound of Formula (I) in an organic solvent (optionally containing one or more wetting agents, one or more emulsifying agents or a mixture of said agents). Suitable organic solvents for use in ECs include aromatic hydrocarbons (such as alkylbenzenes or alkylnaphthalenes, exemplified by SOLVESSO 100, SOLVESSO 150 and SOLVESSO 200; SOLVESSO is a Registered Trade Mark), ketones (such as cyclohexanone or methylcyclohexanone) and alcohols (such as benzyl alcohol, furfuryl alcohol or butanol), N-alkylpyrrolidones (such as N-methylpyrrolidone or N-octylpyrrolidone), dimethyl amides of fatty acids (such as C₈-C₁₀ fatty acid dimethylamide) and chlorinated hydrocarbons. An EC product may spontaneously emulsify on addition to water, to produce an emulsion with sufficient stability to allow spray application through appropriate equipment.

Preparation of an EW involves obtaining a compound of Formula (I) either as a liquid (if it is not a liquid at room temperature, it may be melted at a reasonable temperature, typically below 70°C) or in solution (by dissolving it in an appropriate solvent) and then emulsifying the resultant liquid or solution into water containing one or more SAAs, under high shear, to produce an emulsion. Suitable solvents for use in EWs include vegetable oils, chlorinated hydrocarbons (such as chlorobenzenes), aromatic solvents (such as alkylbenzenes or alkylnaphthalenes) and other appropriate organic solvents which have a low solubility in water.

Microemulsions (ME) may be prepared by mixing water with a blend of one or more solvents with one or more SAAs, to produce spontaneously a thermodynamically stable isotropic liquid formulation. A compound of Formula (I) is present initially in either the water or the solvent/SAA blend. Suitable solvents for use in MEs include those hereinbefore described for use in in ECs or in EWs. An ME may be either an oil-in-water or a water-in-oil system (which system is present may be determined by conductivity measurements) and may be suitable for mixing water-soluble and oil-soluble pesticides in the same formulation. An ME is suitable for dilution into water, either remaining as a microemulsion or forming a conventional oil-in-water emulsion.

Suspension concentrates (SC) may comprise aqueous or non-aqueous suspensions of finely divided insoluble solid particles of a compound of Formula (I). SCs may be prepared by ball or bead milling the solid compound of Formula (I) in a suitable medium, optionally with one or more dispersing agents, to produce a fine particle suspension of the compound. One or more wetting agents may be included in the composition and a suspending agent may be included to reduce the rate at which the particles settle. Alternatively, a compound of Formula (I) may be dry milled and added to water, containing agents hereinbefore described, to produce the desired end product.

Aerosol formulations comprise a compound of Formula (I) and a suitable propellant (for example *n*-butane). A compound of Formula (I) may also be dissolved or dispersed in a suitable medium (for example water or a water miscible liquid, such as n-propanol) to provide compositions for use in non-pressurised, hand-actuated spray pumps.

Capsule suspensions (CS) may be prepared in a manner similar to the preparation of EW formulations but with an additional polymerisation stage such that an aqueous dispersion of oil droplets is obtained, in which each oil droplet is encapsulated by a polymeric shell and contains a compound of Formula (I) and, optionally, a carrier or diluent therefor. The polymeric shell may be produced by either an interfacial polycondensation reaction or by a coacervation procedure. The compositions may provide for controlled release of the compound of Formula (I) and they may be used for seed treatment. A compound of Formula (I) may also be formulated in a biodegradable polymeric matrix to provide a slow, controlled release of the compound.

The composition may include one or more additives to improve the biological performance of the composition, for example by improving wetting, retention or distribution on surfaces; resistance to rain on treated surfaces; or uptake or mobility of a compound of Formula (I). Such additives include surface active agents (SAAs), spray additives based on oils, for example certain mineral oils or natural plant oils (such as soy bean and rape seed oil), modified plant oils such as methylated rape seed oil (MRSO), and blends of these with other bio-enhancing adjuvants (ingredients which may aid or modify the action of a compound of Formula (I).

Wetting agents, dispersing agents and emulsifying agents may be SAAs of the cationic, anionic, amphoteric or non-ionic type.

Suitable SAAs of the cationic type include quaternary ammonium compounds (for example cetyltrimethyl ammonium bromide), imidazolines and amine salts.

Suitable anionic SAAs include alkali metals salts of fatty acids, salts of aliphatic monoesters of sulphuric acid (for example sodium lauryl sulphate), salts of sulphonated aromatic compounds (for example sodium dodecylbenzenesulphonate, calcium dodecylbenzenesulphonate, butyl naphthalene sulphonate and mixtures of sodium di-isopropyl- and tri-isopropyl-naphthalene sulphonates), ether sulphates, alcohol ether sulphates (for example sodium laureth-3-sulphate), ether carboxylates (for example sodium laureth-3-carboxylate), phosphate esters (products from the reaction between one or more fatty alcohols and phosphoric acid (predominately mono-esters) or phosphorus pentoxide (predominately di-esters), for example the reaction between lauryl alcohol and tetraphosphoric acid; additionally these products may be ethoxylated), sulphosuccinamates, paraffin or olefine sulphonates, taurates, lignosulphonates and phosphates / sulphates of tristyrylphenols.

Suitable SAAs of the amphoteric type include betaines, propionates and glycinates.

Suitable SAAs of the non-ionic type include condensation products of alkylene oxides, such as ethylene oxide, propylene oxide, butylene oxide or mixtures thereof, with fatty alcohols (such as oleyl alcohol or cetyl alcohol) or with alkylphenols (such as octylphenol, nonylphenol or octylcresol); partial esters derived from long chain fatty acids or hexitol anhydrides; condensation products of said partial esters with ethylene oxide; block polymers (comprising ethylene oxide and propylene oxide); alkanolamides; simple esters (for example fatty acid polyethylene glycol esters); amine oxides (for example lauryl dimethyl amine oxide); lecithins and sorbitans and esters thereof, alkyl polyglycosides and tristyrylphenols.

Suitable suspending agents include hydrophilic colloids (such as polysaccharides, polyvinylpyrrolidone or sodium carboxymethylcellulose) and swelling clays (such as bentonite or attapulgite).

The herbicidal compounds of present invention can also be used in mixture with one or more additional herbicides and/or plant growth regulators. Examples of such additional herbicides or plant growth regulators include acetochlor, acifluorfen (including acifluorfen-sodium), aclonifen, ametryn, amicarbazone, aminopyralid, aminotriazole, atrazine, beflubutamid-M, bensulfuron (including bensulfuron-methyl), bentazone, bicyclopyrone, bilanafos, bispyribac-sodium, bixlozone, bromacil, bromoxynil, butachlor, butafenacil, carfentrazone (including carfentrazone-ethyl), cloransulam (including cloransulam-methyl), chlorimuron (including chlorimuron-ethyl), chlorotoluron, chlorsulfuron, cinmethylin, clacyfos, clethodim, clodinafop (including clodinafop-propargyl), clomazone, clopyralid, cyclopyranil, cyclopyrimorate, cyclosulfamuron, cyhalofop (including cyhalofop-butyl), 2,4-D (including the choline salt and 2-ethylhexyl ester thereof), 2,4-DB, desmedipham, dicamba (including the aluminium, aminopropyl, bis-aminopropylmethyl, choline, dichloroprop, diglycolamine, dimethylamine, dimethylammonium, potassium and sodium salts thereof) diclosulam, diflufenican, diflufenzopyr, dimethachlor, dimethenamid-P, diquat dibromide, diuron, ethalfluralin, ethofumesate, fenoxaprop (including fenoxaprop-P-ethyl), fenoxasulfone, fenquinotrione, fentrazamide, flazasulfuron, florasulam, florpyrauxifen (including florpyrauxifen-benzyl), fluazifop (including fluazifop-P-butyl), flucarbazone (including flucarbazone-sodium), flufenacet, flumetsulam, flumioxazin, fluometuron, flupyrsulfuron (including flupyrsulfuron-methyl-sodium), fluroxypyr (including fluroxypyr-meptyl), fomesafen, foramsulfuron, glufosinate (including the ammonium salt thereof), glyphosate (including the diammonium, isopropylammonium and potassium salts thereof), halauxifen (including halauxifen-methyl), haloxyfop (including haloxyfop-methyl), hexazinone, hydantocidin, imazamox, imazapic, imazapyr, imazethapyr, indaziflam, iodosulfuron (including iodosulfuron-methyl-sodium), iofensulfuron (including iofensulfuron-sodium), ioxynil, isoproturon, isoxaflutole, lancotrione, MCPA, MCPB, mecoprop-P, mesosulfuron (including mesosulfuron-methyl), mesotrione, metamitron, metazachlor, methiozolin, metolachlor, metosulam, metribuzin, metsulfuron, napropamide, nicosulfuron, norflurazon, oxadiazon, oxasulfuron, oxyfluorfen, paraquat dichloride, pendimethalin, penoxsulam, phenmedipham, picloram, pinoxaden, pretilachlor, primisulfuron-methyl, prometryne, propanil, propaquizafop, propyrisulfuron, propyzamide, prosulfocarb, prosulfuron, pyraclonil, pyraflufen (including pyraflufen-ethyl), pyrasulfotole, pyridate, pyriftalid, pyrimisulfan, pyroxasulfone, pyroxsulam, quinclorac, quinmerac, quizalofop (including quizalofop-P-ethyl and quizalofop-P-tefuryl), rimsulfuron, saflufenacil, sethoxydim, simazine, S-metalochlor, sulfentrazone, sulfosulfuron, tebuthiuron, tefuryltrione, tembotrione, terbuthylazine, terbutryn, tetflupyrolimet, thiencarbazone, thifensulfuron, tiafenacil, tolpyralate, topramezone, tralkoxydim, triafamone, triallate, triasulfuron, tribenuron (including tribenuron-methyl), triclopyr, trifloxysulfuron (including trifloxysulfuron-sodium), trifludimoxazin, trifluralin, triflusulfuron, ethyl 2-[[3-[2-chloro-4-fluoro-5-[3-methyl-2,6-dioxo-4-(trifluoromethyl)pyrimidin-1-yl]phenoxy]-2-pyridyl]oxy]acetate, 3-(2-chloro-4-fluoro-5-(3-methyl-2,6-dioxo-4-trifluoromethyl-3,6-dihydropyrimidin-1 (2H)-yl)phenyl)-5-methyl-4,5-dihydroisoxazole-5-carboxylic acid ethyl ester, 4-hydroxy-1-methoxy-5-methyl-3-[4-(trifluoromethyl)-2-pyridyl]imidazolidin-2-one, 4-hydroxy-1,5-dimethyl-3-[4-(trifluoromethyl)-2-pyridyl]imidazolidin-2-one, 5-ethoxy-4-hydroxy-1-methyl-3-[4-(trifluoromethyl)-2-pyridyl]imidazolidin-2-one, 4-hydroxy-1-methyl-3-[4-(trifluoromethyl)-2-pyridyl]imidazolidin-2-one, 4-hydroxy-1,5-dimethyl-3-[1-methyl-5-(trifluoromethyl)pyrazol-3-yl]imidazolidin-2-one, (4R)1-(5-tert-butylisoxazol-3-yl)-4-ethoxy-5-hydroxy-3-methyl-imidazolidin-2-one, 3-[2-(3,4-dimethoxyphenyl)-6-methyl-3-oxo-pyridazine-4-carbonyl]bicyclo[3.2.1]octane-2,4-dione, 2-[2-(3,4-dimethoxyphenyl)-6-methyl-3-oxo-pyridazine-4-carbonyl]-5-methyl-cyclohexane-1,3-dione, 2-[2-(3,4-dimethoxyphenyl)-6-methyl-3-oxo-pyridazine-4-carbonyl]cyclohexane-1,3-dione, 2-[2-(3,4-dimethoxyphenyl)-6-methyl-3-oxo-pyridazine-4-carbonyl]-5,5-dimethyl-cyclohexane-1,3-dione, 6-[2-(3,4-dimethoxyphenyl)-6-methyl-3-oxo-pyridazine-4-carbonyl]-2,2,4,4-tetramethyl-cyclohexane-1,3,5-trione, 2-[2-(3,4-dimethoxyphenyl)-6-methyl-3-oxo-pyridazine-4-carbonyl]-5-ethyl-cyclohexane-1,3-dione, 2-[2-(3,4-dimethoxyphenyl)-6-methyl-3-oxo-pyridazine-4-carbonyl]-4,4,6,6-tetramethyl-cyclohexane-1,3-dione, 2-[6-cyclopropyl-2-(3,4-dimethoxyphenyl)-3-oxo-pyridazine-4-carbonyl]-5-methyl-cyclohexane-1,3-dione, 3-[6-cyclopropyl-2-(3,4-dimethoxyphenyl)-3-oxo-pyridazine-4-carbonyl]bicyclo[3.2.1]octane-2,4-dione, 2-[6-cyclopropyl-2-(3,4-dimethoxyphenyl)-3-oxo-pyridazine-4-carbonyl]-5,5-dimethyl-cyclohexane-1,3-dione, 6-[6-cyclopropyl-2-(3,4-dimethoxyphenyl)-3-oxo-pyridazine-4-carbonyl]-2,2,4,4-tetramethyl-cyclohexane-1,3,5-trione, 2-[6-cyclopropyl-2-(3,4-dimethoxyphenyl)-3-oxo-pyridazine-4-carbonyl]cyclohexane-1,3-dione, 4-[2-(3,4-dimethoxyphenyl)-6-methyl-3-oxo-pyridazine-4-carbonyl]-2,2,6,6-tetramethyl-tetrahydropyran-3,5-dione, 4-[6-cyclopropyl-2-(3,4-dimethoxyphenyl)-3-oxo-pyridazine-4-carbonyl]-2,2,6,6-tetramethyl-tetrahydropyran-3,5-dione and 4-amino-3-chloro-5-fluoro-6-(7-fluoro-1H-indol-6-yl)pyridine-2-carboxylic acid (including agrochemically acceptable esters thereof, for example, methyl 4-amino-3-chloro-5-fluoro-6-(7-fluoro-1H-indol-6-yl)pyridine-2-carboxylate).

The mixing partners of the compound of Formula (I) may also be in the form of esters or salts, as mentioned e.g. in The Pesticide Manual, Sixteenth Edition, British Crop Protection Council, 2012.

The compound of Formula (I) can also be used in mixtures with other agrochemicals such as fungicides, nematicides or insecticides, examples of which are given in The Pesticide Manual.

The mixing ratio of the compound of Formula (I) to the mixing partner is preferably from 1: 100 to 1000:1.

The mixtures can advantageously be used in the above-mentioned formulations (in which case "active ingredient" relates to the respective mixture of compound of Formula (I) with the mixing partner).

The compounds or mixtures of the present invention can also be used in combination with one or more herbicide safeners. Examples of such safeners include benoxacor, cloquintocet (including cloquintocet-mexyl), cyprosulfamide, dichlormid, fenchlorazole (including fenchlorazole-ethyl), fenclorim, fluxofenim, furilazole, isoxadifen (including isoxadifen-ethyl), mefenpyr (including mefenpyr-diethyl), metcamifen and oxabetrinil.

Particularly preferred are mixtures of a compound of Formula (I) with cyprosulfamide, isoxadifen-ethyl, cloquintocet-mexyl and/or metcamifen.

The safeners of the compound of Formula (I) may also be in the form of esters or salts, as mentioned e.g. in The Pesticide Manual, 16th Edition (BCPC), 2012. The reference to cloquintocet-mexyl also applies to a lithium, sodium, potassium, calcium, magnesium, aluminium, iron, ammonium, quaternary ammonium, sulfonium or phosphonium salt thereof as disclosed in WO 02/34048.

Preferably the mixing ratio of compound of Formula (I) to safener is from 100:1 to 1:10, especially from 20:1 to 1:1.

The present invention still further provides a method of controlling weeds at a locus said method comprising application to the locus of a weed controlling amount of a composition comprising a compound of Formula (I): 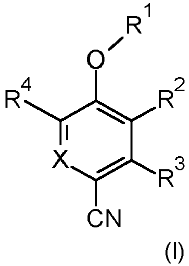 or an agronomically acceptable salt thereof,
wherein
X is CH or N,
R¹ is selected from the group consisting of hydrogen, C₁-C₁₀alkylC(O)-, C₁-C₁₀alkylOC(O)-, C₁-C₁₀alkylSC(O)- and R⁵R⁶NC(O)-;
R² is selected from the group consisting of halogen NO₂, CN, C₁-C₆haloalkylS(O)ₚ-, C₁-C₆alkyl-S(O)ₚ-, C₂-C₄alkenyl-, C₂-C₄alkynyl-, C₁-C₄haloalkyl and C₁-C₄haloalkoxy-;
R³ is selected from the group consisting of hydrogen, halogen, C₁-C₄alkyl, C₁-C₄haloalkyl, C₁-C₄alkoxy- and C₁-C₄haloalkoxy-;
R⁴ is phenyl or a five or six-membered heteroaryl, the heteroaryl containing from one to three heteroatoms each independently selected from the group consisting of oxygen, nitrogen and sulphur, and wherein the phenyl or heteroaryl may be optionally substituted by one or more substituents selected from the group consisting of halogen, C₁-C₆alkyl-, C₂-C₄alkenyl-, C₂-C₄alkynyl-, C₁-C₆haloalkyl-, C₁-C₆alkoxy-, C₁-C₆alkoxyC₁-C₃alkyl-, C₁-C₆alkoxyC₁-C₃alkoxy-, C₁-C₆haloalkoxy-, C₁-C₆alkyl-S(O)ₚ-, C₁-C₆haloalkyl-S(O)ₚ-, C₃-C₆cycloalkyl- (optionally substituted by 1 or 2 halogen), cyano and nitro; or
R⁴ is R4^{a} 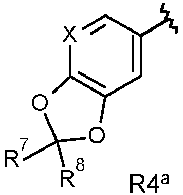 and
R⁵ is hydrogen or C₁-C₁₀alkyl;
R⁶ is hydrogen or C₁-C₁₀alkyl;
R⁷ and R⁸ are independently selected from the group consisting of hydrogen and halogen; and
p = 0, 1 or 2;

The present invention further relates to said method featuring preferred embodiments of compounds of Formula (I) referred to herein.

Moreover, the present invention may further provide a method of selectively controlling weeds at a locus comprising crop plants and weeds, wherein the method comprises application to the locus of a weed controlling amount of a composition according to the present invention. 'Controlling' means killing, reducing or retarding growth or preventing or reducing germination. It is noted that the compounds of the present invention show an improved selectivity compared to know, structurally similar compounds. Generally the plants to be controlled are unwanted plants (weeds). 'Locus' means the area in which the plants are growing or will grow. The application may be applied to the locus pre-emergence and/or postemergence of the crop plant. Some crop plants may be inherently tolerant to herbicidal effects of compounds of Formula (I). Preferred crop plants include maize, wheat, barley and rice.

The rates of application of compounds of Formula I may vary within wide limits and depend on the nature of the soil, the method of application (pre- or postemergence; seed dressing; application to the seed furrow; no tillage application etc.), the crop plant, the weed(s) to be controlled, the prevailing climatic conditions, and other factors governed by the method of application, the time of application and the target crop. The compounds of Formula I according to the invention are generally applied at a rate of from 10 to 2500 g/ha, especially from 25 to 1000 g/ha, more especially from 25 to 250 g/ha.

The application is generally made by spraying the composition, typically by tractor mounted sprayer for large areas, but other methods such as dusting (for powders), drip or drench can also be used.

Crop plants are to be understood as also including those crop plants which have been rendered tolerant to other herbicides or classes of herbicides (e.g. ALS-, GS-, EPSPS-, PPO-, HPPD-, -PDS and ACCase-inhibitors) by conventional methods of breeding or by genetic engineering. An example of a crop that has been rendered tolerant to imidazolinones, e.g. imazamox, by conventional methods of breeding is Clearfield^{®} summer rape (canola). Examples of crops that have been rendered tolerant to herbicides by genetic engineering methods include e.g. glyphosate- and glufosinate-resistant maize varieties commercially available under the trade names RoundupReady^{®} and LibertyLink^{®}.

Crop plants are also to be understood as being those which have been rendered resistant to harmful insects by genetic engineering methods, for example Bt maize (resistant to European corn borer), Bt cotton (resistant to cotton boll weevil) and also Bt potatoes (resistant to Colorado beetle). Examples of Bt maize are the Bt 176 maize hybrids of NK^{®} (Syngenta Seeds). The Bt toxin is a protein that is formed naturally by *Bacillus thuringiensis* soil bacteria. Examples of toxins, or transgenic plants able to synthesise such toxins, are described in EP-A-451 878, EP-A-374 753, WO 93/07278, WO 95/34656, WO 03/052073 and EP-A-427 529. Examples of transgenic plants comprising one or more genes that code for an insecticidal resistance and express one or more toxins are KnockOut^{®} (maize), Yield Gard^{®} (maize), NuCOTIN33B^{®} (cotton), Bollgard^{®} (cotton), NewLeaf^{®} (potatoes), NatureGard^{®} and Protexcta^{®}. Plant crops or seed material thereof can be both resistant to herbicides and, at the same time, resistant to insect feeding ("stacked" transgenic events). For example, seed can have the ability to express an insecticidal Cry3 protein while at the same time being tolerant to glyphosate.

Crop plants are also to be understood to include those which are obtained by conventional methods of breeding or genetic engineering and contain so-called output traits (e.g. improved storage stability, higher nutritional value and improved flavour).

The compositions can be used to control unwanted plants (collectively, 'weeds'). The weeds to be controlled may be both monocotyledonous species, for example *Agrostis, Alopecurus, Avena, Brachiaria, Bromus, Cenchrus, Cyperus, Digitaria, Echinochloa, Eleusine, Lolium, Monochoria, Rottboellia, Sagittaria, Scirpus, Setaria and Sorghum,* and dicotyledonous species, for example *Abutilon, Amaranthus, Ambrosia, Chenopodium, Chrysanthemum, Conyza, Galium, Ipomoea, Nasturtium, Sida, Sinapis, Solanum, Stellaria, Veronica, Viola and Xanthium.*

The present invention further relates to the use of a compound of Formula (I): 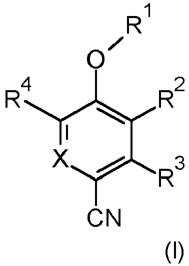 or an agronomically acceptable salt thereof,
wherein
X is CH or N,
R¹ is selected from the group consisting of hydrogen, C₁-C₁₀alkylC(O)-, C₁-C₁₀alkylOC(O)-, C₁-C₁₀alkylSC(O)- and R⁵R⁶NC(O)-;
R² is selected from the group consisting of halogen NO₂, CN, C₁-C₆haloalkylS(O)ₚ-, C₁-C₆alkyl-S(O)ₚ-, C₂-C₄alkenyl-, C₂-C₄alkynyl-, C₁-C₄haloalkyl and C₁-C₄haloalkoxy-;
R³ is selected from the group consisting of hydrogen, halogen, C₁-C₄alkyl, C₁-C₄haloalkyl, C₁-C₄alkoxy- and C₁-C₄haloalkoxy-;
R⁴ is phenyl or a five or six-membered heteroaryl, the heteroaryl containing from one to three heteroatoms each independently selected from the group consisting of oxygen, nitrogen and sulphur, and wherein the phenyl or heteroaryl may be optionally substituted by one or more substituents selected from the group consisting of halogen, C₁-C₆alkyl-, C₂-C₄alkenyl-, C₂-C₄alkynyl-, C₁-C₆haloalkyl-, C₁-C₆alkoxy-, C₁-C₆alkoxyC₁-C₃alkyl-, C₁-C₆alkoxyC₁-C₃alkoxy-, C₁-C₆haloalkoxy-, C₁-C₆alkyl-S(O)ₚ-, C₁-C₆haloalkyl-S(O)ₚ-, C₃-C₆cycloalkyl- (optionally substituted by 1 or 2 halogen), cyano and nitro; or
R⁴ is R4^{a} 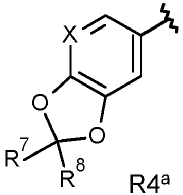 and
R⁵ is hydrogen or C₁-C₁₀alkyl;
R⁶ is hydrogen or C₁-C₁₀alkyl;
R⁷ and R⁸are independently selected from the group consisting of hydrogen and halogen; and
p = 0, 1 or 2;
as a herbicide.

The present invention further relates to said use featuring preferred embodiments of compounds of Formula (I) referred to herein.

The compounds of the present invention can be prepared according to the following general schemes.

(5-cyano-2-methoxy-phenyl)boronic acid can undergo cross-coupling with the desired aryl or heteroaryl halide (X can be I, Br, CI) under palladium catalysis, employing a suitable palladium salt, ligand, base and solvent under elevated temperature. The resulting product is subjected to demethylation conditions, such as boron tribromide in a suitable solvent such as dichloromethane. The phenol can be halogenated with a suitable halogenating reagent such as N-bromosuccinimide at ambient or elevated temperatures, in a suitable solvent such as acetonitrile. The phenol can be further reacted with a range of acid chlorides, anhydrides or activated esters to afford the phenol esters.

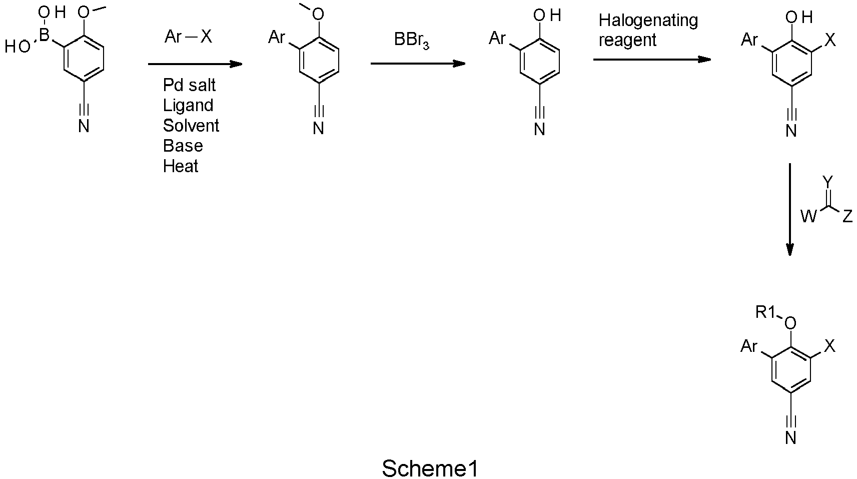

Alternatively, the desired substituted phenol (Scheme 2) can be halogenated with a suitable halogenating reagent such as N-bromosuccinimide, either at ambient or elevated temperatures in a suitable solvent such as acetonitrile. The halogenated phenol can undergo cross-coupling with the desired aryl or heteroaryl boronic acid or ester, under palladium catalysis, employing a suitable palladium salt, ligand, base and solvent under elevated temperature (*Path A*). Where R² is also a halogen, isomeric mixtures may result which can be separated by chromatography. These phenols can be further reacted with a range of acid chlorides, anhydrides or activated esters to afford the phenol esters. Alternatively, the halogenated phenol can be reacted with the desired acid chloride, anhydride or activated ester to afford the phenol esters (*Path B*). These intermediates can undergo cross-coupling with the desired aryl or heteroaryl boronic acid or ester, under palladium catalysis, employing a suitable palladium salt, ligand, base and solvent under elevated temperature to afford further compounds of the invention.

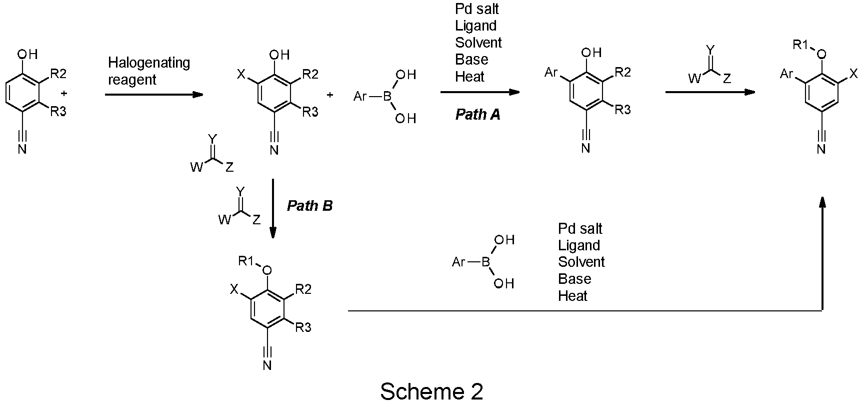

### General

Alternatively, the desired substituted phenol (Scheme 3) can be halogenated with a suitable halogenating reagent such as N-bromosuccinimide, either at ambient or elevated temperatures in a suitable solvent such as acetonitrile. The halogenated phenol can then be protected with a suitable protecting group, such a benzyl or paramethoxybenzyl group, using benzyl chloride or paramethoxybenzylchloride, with a suitable base, such as potassium carbonate, in a suitable solvent such as acetone with a phase transfer catalyst such as tetrabutylammonium iodide. In a following step the bromide undergoes metalation with a Grignard reagent at low temperature in a suitable solvent such as THF. The resulting aryl Grignard is then reacted with a desired boronate, such as 2-ethoxy-4,4,5,5-tetramethyl-1,3,2-dioxaborolane. The resulting aryl boronate can then be coupled to a range of aryl or heteroaryl halides in a Suzuki reaction, under elevated temperature, followed by deprotection, to afford further compounds of the invention.

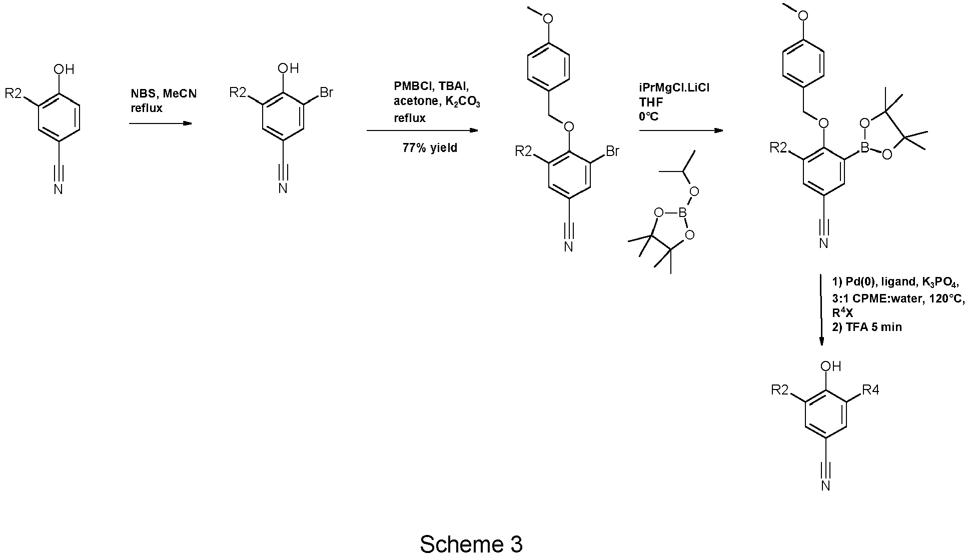

### Example 1. Synthesis of 3-bromo-4-hydroxy-5-[1-methyl-5-(trifluoromethyl)pyrazol-3-yl]benzonitrile - Compound 1.007

### Step 1 Synthesis of 4-methoxy-3-[1-methyl-5-(trifluoromethyl)pyrazol-3-yl]benzonitrile

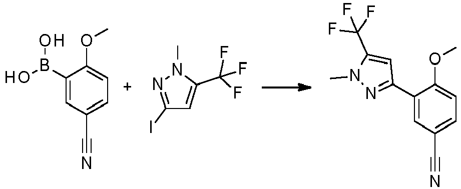

A 25mL round bottomed flask was charged with a stirrer bar, (5-cyano-2-methoxyphenyl)boronic acid (4.3 mmol, 0.76 g), 3-iodo-1-methyl-5-(trifluoromethyl)pyrazole (4 mmol, 1.10 g) and bis(triphenylphosphine)palladium dichloride (0.4 mmol, 0.28g). Acetonitrile (10mL) was added followed by and potassium carbonate (6 mmol, 0.82927 g) and mixture was heated at reflux. After 3h, the reaction was diluted with dichloromethane (15mL), washed with and brine (10mL), dried with MgSO₄ and concentrated. The crude product was then purified by chromatography (gradient elution EtOAc/Hexane) to afford 4-methoxy-3-[1-methyl-5-(trifluoromethyl)pyrazol-3-yl]benzonitrile (665mg, 60%) as an off white solid.

¹HNMR (CDCl₃) δ 8.28 (d, 1H), 7.51 (dd, 1H), 7.12 (s, 1H), 7.02(1H, d), 5.04(3H, s), 3.59 (3H, s).

### Step 2 Synthesis of 4-hydroxy-3-[1-methyl-5-(trifluoromethyl)pyrazol-3-yl]benzonitrile

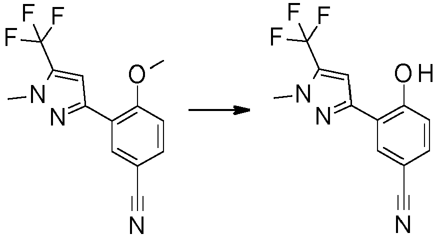

To a solution of 4-methoxy-3-[1-methyl-5-(trifluoromethyl)pyrazol-3-yl]benzonitrile (0.65 g, 2.312 mmol) in anhydrous dichloromethane (20mL) at 0°C and under nitrogen was added boron tribromide (1M solution in DCM) (8.090 mL, 8.090 mmol) dropwise. Once addition was complete, the reaction mixture was allowed to warm to room temperature and stirred for a further 2h. The reaction was carefully quenched with water and then extracted with dichloromethane (3x10mL). The organics were combined, washed with brine, dried over magnesium sulfate and concentrated in vacuo to afford 4-hydroxy-3-[1-methyl-5-(trifluoromethyl)pyrazol-3-yl]benzonitrile yellow solid (600mg, 97%).

¹HNMR (CDCl₃) δ 10.80 (s, 1H), 7.82 (d, 1H), 7.51 (dd, 1H), 7.09(1H, d), 7.01(1H, s), 4.08 (3H, s).

### Step 3 Synthesis of 3-bromo-4-hydroxy-5-[1-methyl-5-(trifluoromethyl)pyrazol-3-yl]benzonitrile - Compound 1.007

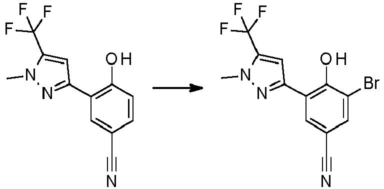

To a stirred solution of 4-hydroxy-3-[1-methyl-5-(trifluoromethyl)pyrazol-3-yl]benzonitrile (0.580g, 2.1706 mmol,) in acetonitrile (10 mL) was added 1-bromopyrrolidine-2,5-dione (0.579g, 3.25 mmol), in one portion, and the mixture was heated at reflux for 2h. The mixture was then diluted with EtOAc and washed with water (10mL) and Brine (10), dried (MgSO4) and purified by chromatography to afford 3-bromo-4-hydroxy-5-[1-methyl-5-(trifluoromethyl)pyrazol-3-yl]benzonitrile (450mg, 60%).

¹HNMR (CDCl₃) δ 11.59 (s, 1H), 7.80 (s, 1H), 7.36 (s, 1H), 7.03 (s, 1H), 4.09 (3H, s).

### Example 2. Synthesis of 4-bromo-6-(3-fluorophenyl)-5-hydroxy-pyridine-2-carbonitrile (Compound 1.040) and 6-bromo-4-(3-fluorophenyl)-5-hydroxypyridine-2-carbonitrile (Compound 1.039).

### Step 1 Synthesis of (2,4-dibromo-6-cyano-3-pyridyl) acetate

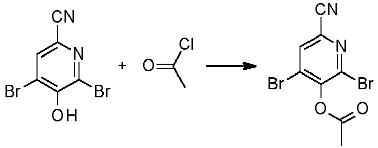

4,6-dibromo-5-hydroxy-pyridine-2-carbonitrile (1 g, 3.5984 mmol), dichloromethane (10 mL) N,N-diethylethanamine (1.2 equiv., 4.3181 mmol) were combined in a 50mL round bottomed flask and acetyl chloride (1.1 equiv., 3.9583 mmol) was added dropwise. After stirring at room temperature for 5h, the mixture was washed with water (15mL) and brine (15mL), dried (MgSO4) and concentrated to afford a 2,4-dibromo-6-cyano-3-pyridyl) acetate 1.07g (93%).

¹HNMR (CDCl₃) δ 7.91 (s,1H), 2.46 (s, 3H).

### Step 2 Synthesis of 4-bromo-6-(3-fluorophenyl)-5-hydroxy-pyridine-2-carbonitrile and 6-bromo-4-(3-fluorophenyl)-5-hydroxy-pyridine-2-carbonitrile

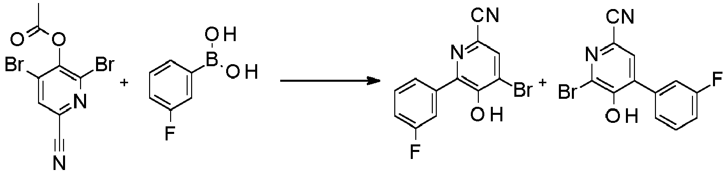

(2,4-dibromo-6-cyano-3-pyridyl) acetate (0.2 g, 0.625mmol), (3-fluorophenyl)boronic acid (87mg 0.625 mmol,) and bis(triphenylphosphine)palladium(ll)dichloride (22mg, 0.031 mmol) were weighed into a microwave vial. Acetonitrile (5mL) was added followed by a stirrer bar and sodium carbonate (1.2502 mmol). The vial was sealed and heated for 10 min at 130°C in the microwave. The mixture was diluted with EtOAc (15mL) and washed with 2M HCl (10mL), and brine (10mL), dried with MgSO₄ and concentrated. The resulting crude product was purified by RPHPLC (to afford the separated isomers 4-bromo-6-(3-fluorophenyl)-5-hydroxy-pyridine-2-carbonitrile (15mg) and 6-bromo-4-(3-fluorophenyl)-5-hydroxy-pyridine-2-carbonitrile (10mg).

4-bromo-6-(3-fluorophenyl)-5-hydroxy-pyridine-2-carbonitrile ¹HNMR (DMSO-d₆) δ 8.31 (s,1H), 7.73 (t, 2H), 7.53 (q, 1H), 7.29 (td, 1H).

6-bromo-4-(3-fluorophenyl)-5-hydroxy-pyridine-2-carbonitrile ¹HNMR (DMSO-d₆) δ 7.98 (s,1H), 7.58-7.48 (m, 3H), 7.28-7.24 (m, 1H).

### Example 3. Synthesis of 3-bromo-5-(3-fluorophenyl)-4-hydroxy-benzonitrile - Compound 1.046.

### Step 1 Synthesis of (2,6-dibromo-4-cyano-phenyl) acetate

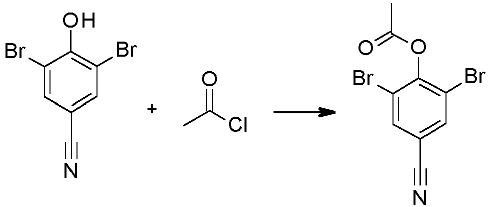

3,5-dibromo-4-hydroxy-benzonitrile (5 g, 18.05 mmol) was weighed into a 250ml round bottom flask and a stirrer bar added. Dichloromethane (100 mL) was added followed by triethylamine (1.1 equiv, 19.862 mmol) and the flask was cooled in an ice-bath. Acetyl chloride (1.1 equiv., 19.862 mmol) was added dropwise into the reaction mixture which was stirred for 1h. The reaction mixture was washed with water (100ml × 3) and the organic phase dried (MgSO4). The mixture was concentrated, in vacuo to afford the desired product (5.5g, 96%).

¹HNMR (CDCl₃) δ 7.88 (s, 2H), 2.42 (s, 3H).

### Step 2 Synthesis of 3-bromo-5-(3-fluorophenyl)-4-hydroxy-benzonitrile

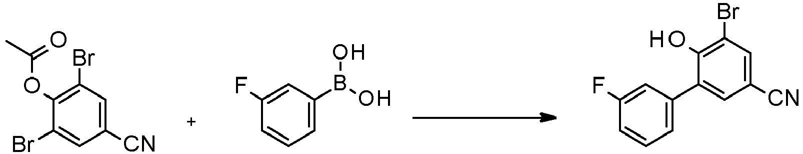

To a microwave tube, charged with a stirrer bar, was added (3-fluorophenyl)boronic acid (91mg, 0.65mmol), bis(triphenylphosphine)palladium (II) dichloride) (0.05 equiv., 0.0325 mmol), and (2,6-dibromo-4-cyano-phenyl) acetate (207mg, 0.65mmol) in acetonitrile (5mL). A 1 molar solution of Sodium carbonate (2 eq. 1.3mmol, 1.3mL), was added and the tube was capped and microwaved at 150°C for 15 mins. The reaction mixture was passed through a SiTMT SPE cartridge. The mixture was then concentrated in vacuo and the residue re-dissolved in 10% MeOH in DMSO and purified by mass directed RPHPLC to afford the 3-bromo-5-(3-fluorophenyl)-4-hydroxy-benzonitrile (32mg, 15%).

¹HNMR (CDCl₃) δ 7.85 (d, 1H), 7.53 (1H, d), 7.50-7.46 (1H, m), 7.27-7.18 (3H, m), 6.20 (1H, bs).

### Example 4. Synthesis of 3-chloro-4-hydroxy-5-[4-(trifluoromethyl)pyrazol-1-yl]benzonitrile - Compound 1.001.

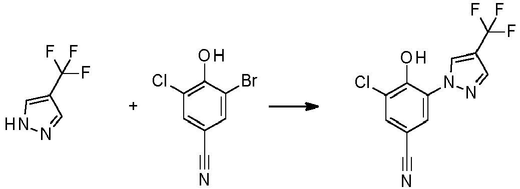

To a stirred solution of 3-bromo-5-chloro-4-hydroxy-benzonitrile (0.100 g, 0.430 mmol) in anhydrous N,N-dimethylformamide (3.00 mL) , 4-(trifluoromethyl)-1H-pyrazole (0.0820 g, 0.602 mmol), Cesium carbonate (0.280 g, 0.860 mmol) and Copper iodide (0.0164 g, 0.0860 mmol) were added at RT. The reaction mixture was purged with nitrogen for 15 min and then heated at 120°C in a sealed tube for 24 hrs. Then the reaction mass was diluted with EtOAc and passed through the bed of silica gel and concentrated to afford the crude product. Purification by chromatography, eluting with 0 to 20% EtOAc in hexane, affords 3-chloro-4-hydroxy-5-[4-(trifluoromethyl)pyrazol-1-yl]benzonitrile (10mg, 8%).

¹HNMR (400MHz,DMSO): δ 8.93 (s, 1H), 8.27 (s, 1H), 8.11-8.10 (m, 2H).

### Example 5. Synthesis of 4-hydroxy-3-thiazol-2-yl-5-(trifluoromethyl)benzonitrile

### Step 1 Synthesis of 3-bromo-4-hydroxy-5-(trifluoromethyl)benzonitrile

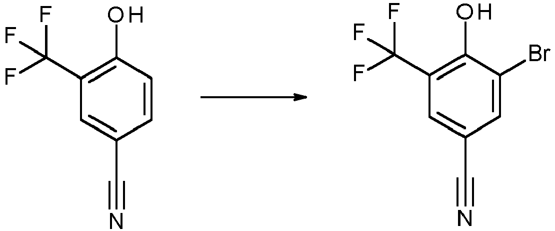

To a stirred solution of 4-hydroxy-3-(trifluoromethyl)benzonitrile (4.5 g, 24 mmol,) in acetonitrile (30 mL) was added N-bromosuccinimide (4.3 g, 24 mmol), in one portion, and the mixture was heated at reflux for 1.5h. The mixture was allowed to cool to room temperature and then dry-loaded on silica and purified by chromatography using a EtOAc/cyclohexane gradient elution to afford 3-bromo-4-hydroxy-5-(trifluoromethyl)benzonitrile (6.4 g).

¹H NMR (400 MHz, chloroform) δ ppm 7.99 (d, *J*=1.83 Hz, 1 H) 7.85 (d, *J*=1.34 Hz, 1 H) 6.34 - 6.90 (br s, 1 H).

### Step 2 Synthesis of 3-bromo-4-[(4-methoxyphenyl)methoxy]-5-(trifluoromethyl) benzonitrile

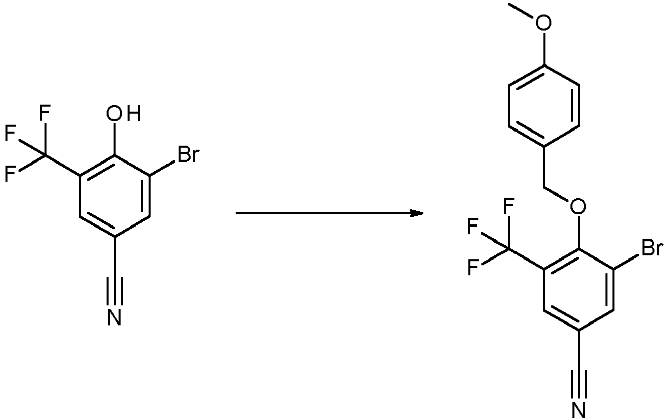

To a solution of 3-bromo-4-hydroxy-5-(trifluoromethyl)benzonitrile (6.4 g, 24 mmol) in acetone (64 mL) were added potassium carbonate (6.7 g, 48 mmol), tetrabutylammonium iodide (1.8 g, 4.8 mmol) and 1-(chloromethyl)-4-methoxybenzene (5.7 g, 36 mmol). The reaction was heated at reflux. After 4h the reaction was worked up with water and ethyl acetate were added and the phases separated. The organic phase was dried over magnesium sulphate and concentrated *in vacuo.* Purification by column chromatography (10% ethyl acetate in cyclohexane) afforded 3-bromo-4-[(4-methoxyphenyl)methoxy]-5-(trifluoromethyl)benzonitrile (77%, 7.18 g)

¹H NMR (400 MHz, chloroform) δ ppm 8.05 - 8.15 (m, 1 H) 7.90 (d, *J*=1.59 Hz, 1 H) 7.40 - 7.54 (m, 2 H) 6.91 - 6.99 (m, 2 H) 5.09 (s, 2 H) 3.84 (s, 3 H).

### Step 3 Synthesis of 4-[(4-methoxyphenyl)methoxy]-3-(4,4,5,5-tetramethyl-1,3,2-dioxaborolan-2-yl)-5-(trifluoromethyl)benzonitrile

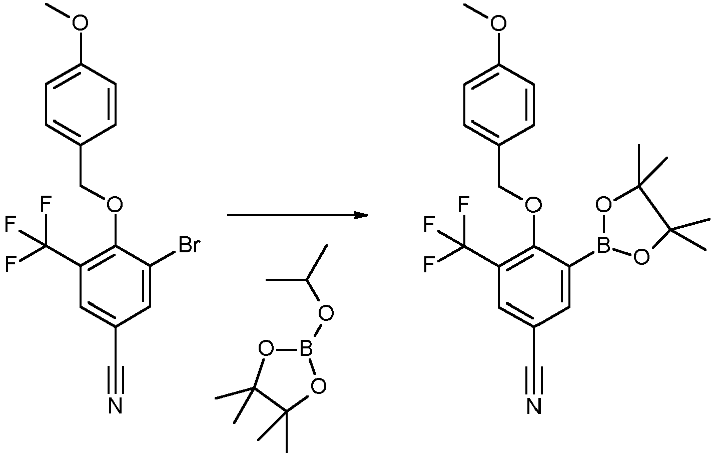

To a stirring solution of 3-bromo-4-[(4-methoxyphenyl)methoxy]-5-(trifluoromethyl)benzonitrile (2.23 g, 5.77 mmol) and 2-isopropoxy-4,4,5,5-tetramethyl-1,3,2-dioxaborolane (1.61 g, 8.66 mmol) in tetrahydrofuran (46.2 mL) at 0°C was added isopropylmagnesium chloride Lithium chloride complex (1.3M in THF) (5.3 mL, 6.93 mmol) (added in a steady stream); after 45 min the reaction was quenched with sat. aq. ammonium chloride solution and extracted into ethyl acetate. The organic phase was dried over magnesium sulphate and concentrated *in vacuo* to afford a 3.4:1 ratio of 4-[(4-methoxyphenyl)methoxy]-3-(4,4,5,5-tetramethyl-1,3,2-dioxaborolan-2-yl)-5-(trifluoromethyl)benzonitrile and deborylated 4-[(4-methoxyphenyl)methoxy]-3-(trifluoromethyl)benzonitrile (3.13 g).

¹H NMR (400 MHz, chloroform) δ ppm 8.24 - 8.34 (m, 1 H) 7.93 - 8.05 (m, 1 H) 7.43 - 7.48 (m, 2 H) 6.90 - 6.95 (m, 2 H) 5.09 (s, 2 H) 3.83 (s, 3 H) 1.39 (s, 12 H).

### Step 4 Synthesis of 4-hydroxy-3-thiazol-2-yl-5-(trifluoromethyl) benzonitrile

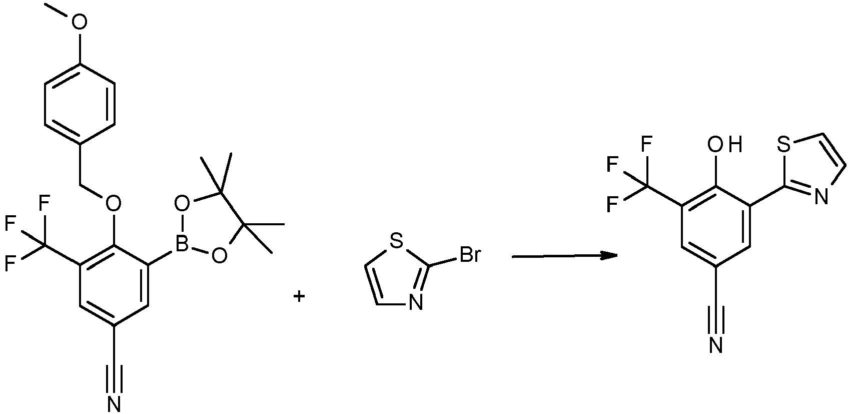

2-bromothiazole (0.046 g, 0.28 mmol), 4-[(4-methoxyphenyl)methoxy]-3-(4,4,5,5-tetramethyl-1,3,2-dioxaborolan-2-yl)-5-(trifluoromethyl)benzonitrile (crude material, assumed -60% purity, 0.17 g, 0.24 mmol), SPhos Pd G3 (0.019 g, 0.024 mmol), tripotassium phosphate trihydrate (0.076 g, 0.35 mmol), cyclopentyl methyl ether (1.7 mL) and water (0.85 mL) were combined in a microwave vial. The vial was sealed and heated in the microwave at 120°C for 10 mins. The sample was then diluted with water/ethyl acetate, the phases were separated, and the organic phase was dried over magnesium sulphate and concentrated *in vacuo.* 2,2,2-trifluoroacetic acid (0.5 mL) was added to the residue and the mixture was stirred for 5 min; water and ethyl acetate were added, the phases were separated and the organic phase was dried over magnesium sulphate and concentrated *in vacuo.* The cured mixture was purified by RPHPLC to afford 4-hydroxy-3-thiazol-2-yl-5-(trifluoromethyl)benzonitrile (0.0142 g, 22%).

¹H NMR (400 MHz, chloroform) δ ppm 8.11 (d, *J*=1.96 Hz, 1 H) 7.92 (d, *J*=3.42 Hz, 1 H) 7.89 (d, *J*=1.34 Hz, 1 H) 7.50 (d, *J*=3.30 Hz, 1 H)

The following tables provide examples of herbicidal compounds of the present invention. Mass spectrometry data was collected on a Waters Acquity UPLC-MS using a Sample Organizer with Sample Manager FTN, H-Class QSM, Column Manager, 2 x Column Manager Aux, Photodiode Array (Wavelength range (nm): 210 to 400), ELSD and QDa mass spectrometer. Ionisation method: Electrospray positive and negative: Cone (V) 20.00, Source Temperature (°C) 120, Cone Gas Flow (L/Hr.) 50. Mass range (Da): positive 100 to 800, negative 115 to 800.

Method 1) analysis was conducted using a four-minute run time, according to the following gradient table at 40°C:

### Waters Acquity UPLC HSS T3 (1.8µm 2.1×50mm).

| Time (mins) | Solvent A (%) | Solvent B (%) | Flow (ml / mn) |
|---|---|---|---|
| 0.00 | 95.0 | 5.0 | 0.6 |
| 3.00 | 0.0 | 100 | 0.6 |
| 3.50 | 0.0 | 100 | 0.6 |
| 3.55 | 95.5 | 5.0 | 0.6 |
| 4.10 | 95.5 | 5.0 | 0.6 |

| | | | |
|---|---|---|---|
| Solvent A: H₂O with 0.05% TFA Solvent B: CH₃CN with 0.05% TFA | | | |

Method 2) Analysis was conducted using a two-minute run time, according to the following gradient table at 40°C:

### Waters Acquity UPLC HSS T3 (1.8µm 2.1x30mm).

| Time (mins) | Solvent A (%) | Solvent B (%) | Flow (ml / mn) |
|---|---|---|---|
| 0.00 | 95.0 | 5.0 | 0.7 |
| 1.75 | 0.0 | 100 | 0.7 |
| 1.76 | 0.0 | 100 | 0.7 |
| 2.00 | 0.0 | 100 | 0.7 |
| 2.01 | 95.5 | 5.0 | 0.7 |
| 2.11 | 95.5 | 5.0 | 0.7 |

| | | | |
|---|---|---|---|
| Solvent A: H₂O with 0.05% TFA Solvent B: CH₃CN with 0.05% TFA | | | |

**Table 1 - Examples of herbicidal compounds of the present invention.**

| **Compound** | **Structure** | **¹HNMR** | **Retention Time** | **MH+/M-H (observed)** |
|---|---|---|---|---|
| 1.001 | 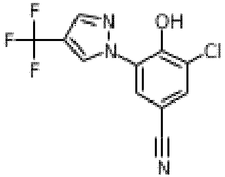 | (DMSO-d₆) δ 8.93 (s, 1H), 8.27 (s, 1H), 8.11-8.10 (m, 2H) | | |
| 1.002 | 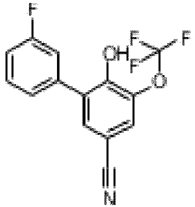 | (DMSO-d₆) δ 11.34 (s, 1H), 7.95 (s 1H), 7.84 (d, 1H), 7.53-7.49 (m, 1H), 7.44-7.41 (m, 2H), 7.27-7.22 (m, 1H). | | |
| 1.003 | 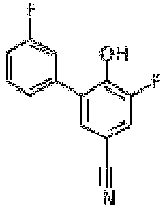 | (DMSO-d₆) δ 1116 (s, 1H), 7.85-7.82 (dd, 1H), 7.67 (s, 1H), 7.52-7.41 (m, 3H), 7.25-7.21 (m, 1H) | | |
| 1.004 | 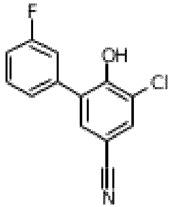 | (DMSO-d₆) δ 10.68 (s, 1H), 800 (d, 1H), 7.75 (d, 1H), 7.52-7.47 (q, 1H), 7.40-7.37 (m, 2H), 7.26-7.22 (m, 1H) | | |
| 1.005 | 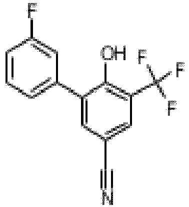 | (DMSO-d₆) δ 1089 (s, 1H), 8.11-8.10 (d, 1H), 7.97-7.96 (d, 1H), 7.56-7.50 (m, 1H), 7.37-7.25 (m, 3H). | | |
| 1.006 | 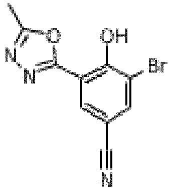 | (DMSO-d₆) 8.48 (d, 1H), 8.24 (d, 1H), 262 (s, 3H) | | |
| 1.007 | 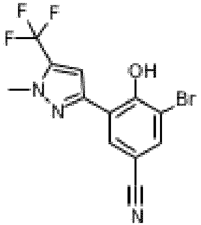 | (CDCl₃) δ 11.59 (s, 1H), 7.80 (s, 1H), 7.26 (s, 1H), 7.03 (s, 1H), 4.09 (3H, s). | | |
| 1.008 | 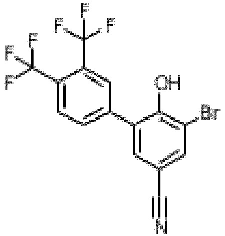 | | 2.65 | 408.1 |
| 1.009 | 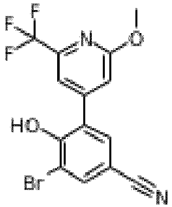 | | 2.49 | 371.1 |
| 1.010 | 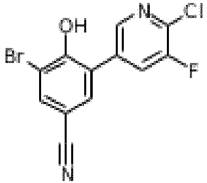 | | 2.16 | 325.0 |
| 1.011 | 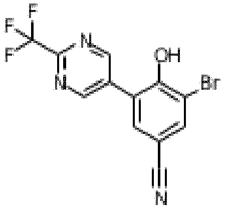 | | 2.06 | 345.0 |
| 1.012 | 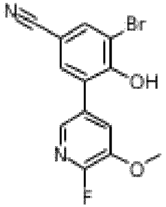 | | 1.99 | 325.0 |
| 1.013 | 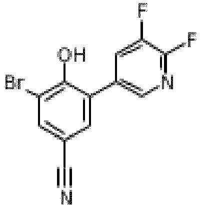 | | 2.08 | 309.9 |
| 1.014 | 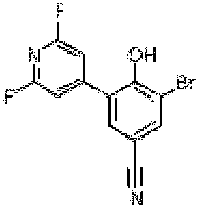 | | 2.13 | 309.0 |
| 1.015 | 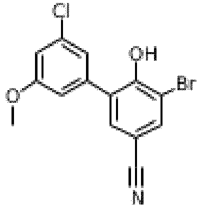 | | 2.47 | 338.1 |
| 1.016 | 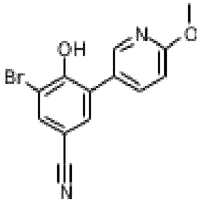 | | 1.94 | 305.0 |
| 1.017 | 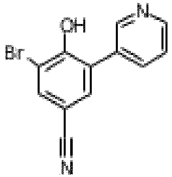 | | 1.12 | 274.99 |
| 1.018 | 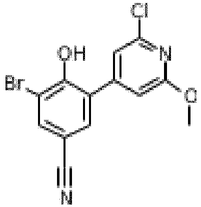 | | 2.36 | 337.1 |
| 1.019 | 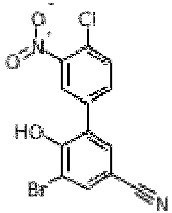 | | 2.34 | 351.0 |
| 1.020 | 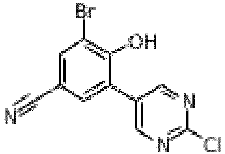 | | 1.4 | 325.0 |
| 1.021 | 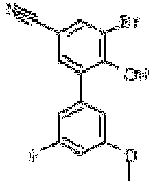 | | 1.85 | 310.0 |
| 1.022 | 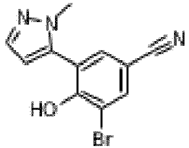 | | 2.32 | 322.0 |
| 1.023 | 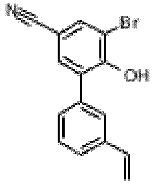 | | 1.64 | 278.0 |
| 1.024 | 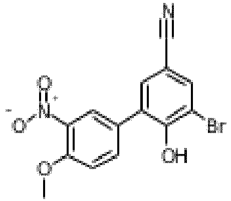 | | 2.17 | 347.1 |
| 1.025 | 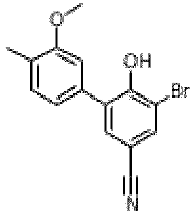 | | 2.45 | 318.1 |
| 1.026 | 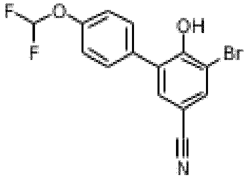 | | 2.33 | 338.1 |
| 1.027 | 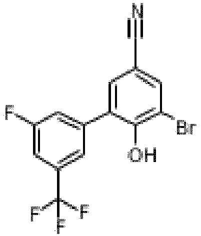 | | 2.51 | 358.1 |
| 1.028 | 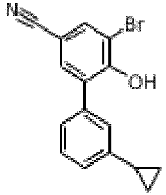 | | 2.52 | 314.1 |
| 1.029 | 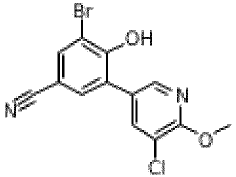 | | 2.31 | 339.0 |
| 1.030 | 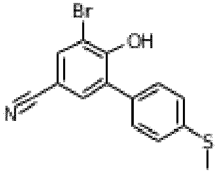 | | 2.41 | 320.2 |
| 1.031 | 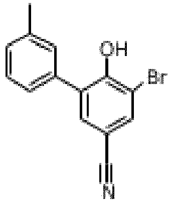 | | 2.39 | 288.6 |
| 1.032 | 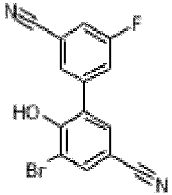 | | 2.17 | 315.1 |
| 1.033 | 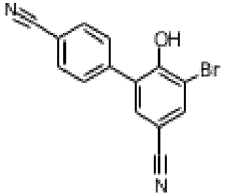 | | 2.09 | 297.1 |
| 1.034 | 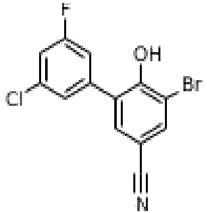 | | 2.46 | 326.0 |
| 1.035 | 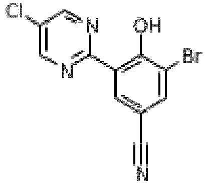 | (DMSO-d₆) δ 14.36 (s, 1H), 9.19 (s, 2H), 8.68 (d, 1H), 8.32 (d, 1H) | | |
| 1.036 | 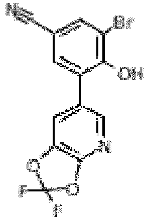 | | | |
| 1.037 | 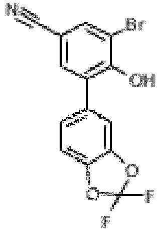 | | | |
| 1.038 | 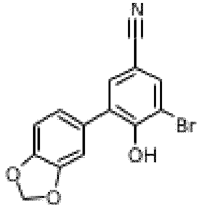 | | | |
| 1.039 | 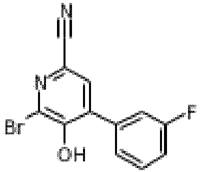 | (DMSO-d₆) δ 7.98 (s, 1H), 7.58-7.48 (m, 3H), 7.28-7.24 (m,1H). | | |
| 1.040 | 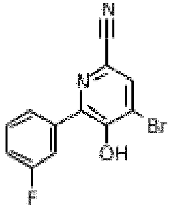 | (DMSO-d₆) δ 8.31 (s, 1H), 7.73 (t, 2H), 7.53 (q, 1H), 7.29 (td, 1H). | | |
| 1.041 | 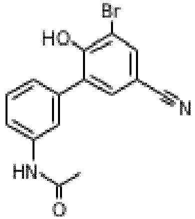 | | 1.42 | 386.4 |
| 1.042 | 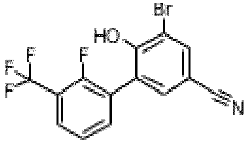 | | 1.41 | 360.2 |
| 1.043 | 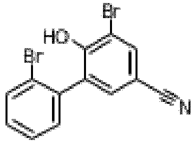 | | 1.35 | 352.1 |
| 1.044 | 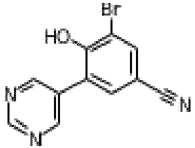 | | 0.87 | 276.2 |
| 1.045 | 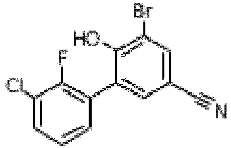 | | 1.37 | 326.1 |
| 1.046 | 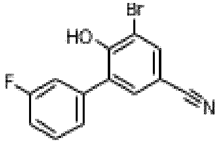 | (CDCl₃) δ 7.85 (d, 1H), 7.53 (d, 1H), 7.50-7.46 (m , 1H), 7.27-7.18 (m 3H), 6.20 (bs, 1H). | | |
| 1.047 | 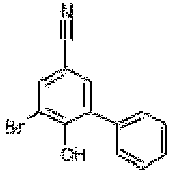 | | 1.31 | 274.2 |
| 1.048 | 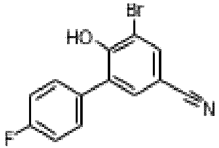 | | 2.27 | 292.2 |
| 1.049 | 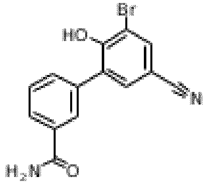 | | 0.98 | 317.2 |
| 1.050 | 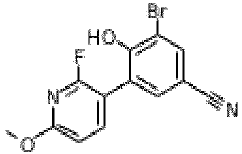 | | 2.5 | 323.2 |
| 1.051 | 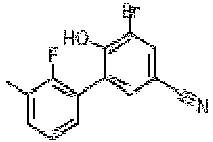 | | 1.37 | 306.2 |
| 1.052 | 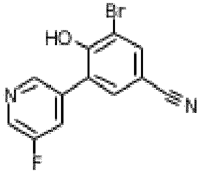 | | 2.26 | 293.1 |
| 1.053 | 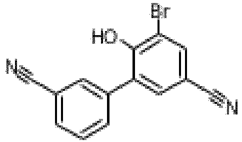 | | 1.23 | 299.2 |
| 1.054 | 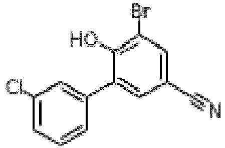 | | 1.41 | 308.2 |
| 1.055 | 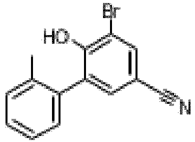 | | 1.36 | 288.2 |
| 1.056 | 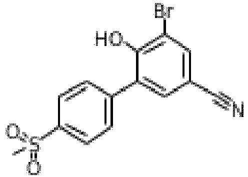 | | 2.73 | 352.1 |
| 1.057 | 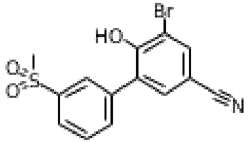 | | 2.61 | 352.3 |
| 1.058 | 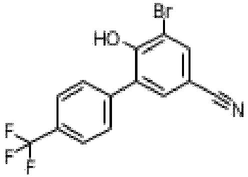 | | 1.45 | 342.2 |
| 1.059 | 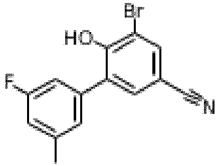 | | 1.42 | 306.2 |
| 1.060 | 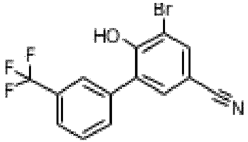 | | 1.44 | 342.2 |
| 1.061 | 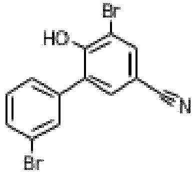 | | 1.43 | 352.1 |
| 1.062 | 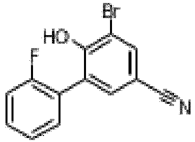 | | 1.28 | 292.2 |
| 1.063 | 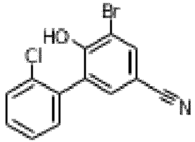 | | 1.33 | 308.1 |
| 1.064 | 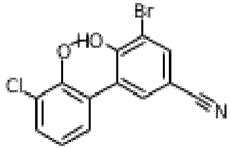 | | 1.38 | 338.2 |
| 1.065 | 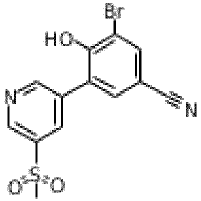 | | 2.29 | 353.1 |
| 1.066 | 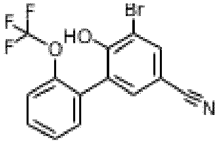 | | 1.39 | 358.2 |
| 1.067 | 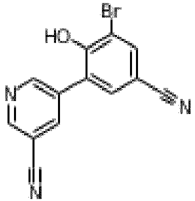 | | 1.76 | 300.0 |
| 1.068 | 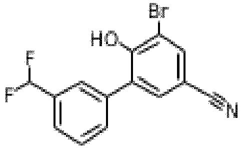 | | 1.34 | 324.2 |
| 1.069 | 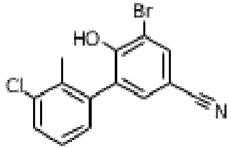 | | 1.46 | 322.2 |
| 1.070 | 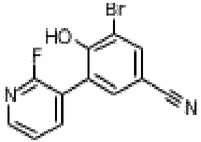 | | 0.93 | 293.1 |
| 1.071 | 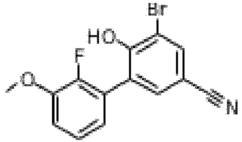 | | 1.28 | 322.1 |
| 1.072 | 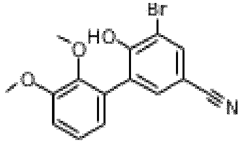 | | 1.3 | 391.2 |
| 1.073 | 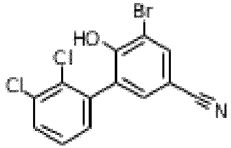 | | 1.42 | 342.1 |
| 1.074 | 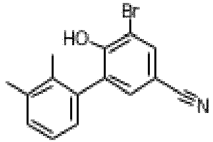 | | 1.44 | 302.1 |
| 1.075 | 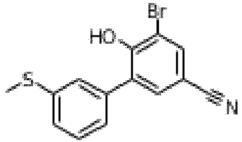 | | 1.41 | 320.2 |
| 1.076 | 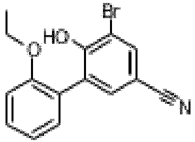 | | 1.39 | 318.2 |
| 1.077 | 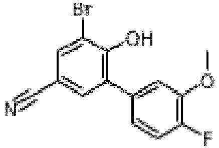 | | | |
| 1.078 | 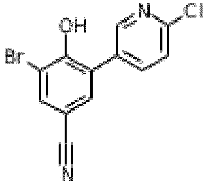 | | 1.98 | 307.0 |
| 1.079 | 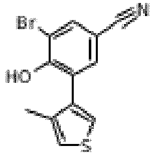 | | 2.24 | 293.8 |
| 1.080 | 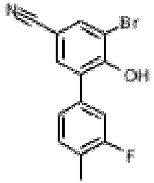 | | 2.39 | 306.1 |
| 1.081 | 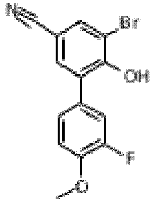 | | 2.23 | 320.1 |
| 1.082 | 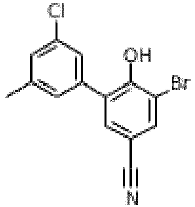 | | 2.53 | 322.0 |
| 1.083 | 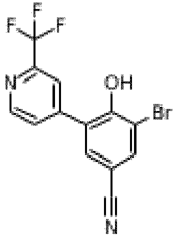 | | 2.11 | 343.0 |
| 1.084 | 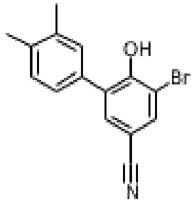 | | 2.50 | 302.0 |
| 1.085 | 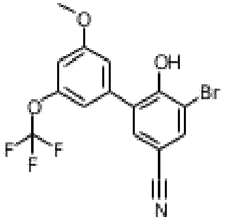 | | 2.54 | 386.1 |
| 1.086 | 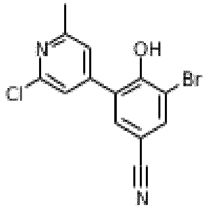 | | 2.08 | 323.0 |
| 1.087 | 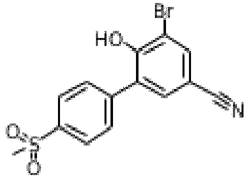 | | 1.82 | 350.0 |
| 1.088 | 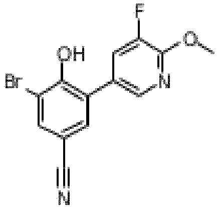 | | 2.14 | 321.0 |
| 1.089 | 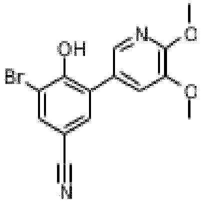 | | 1.96 | 333.0 |
| 1.090 | 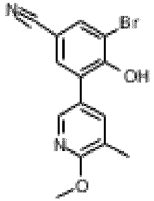 | | 2.20 | 317.1 |
| 1.091 | 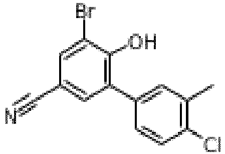 | | 2.54 | 322.0 |
| 1.092 | 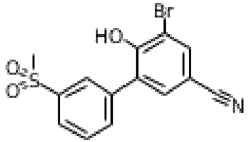 | | 1.82 | 352.0 |
| 1.093 | 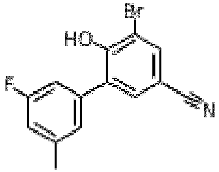 | | 2.39 | 306.0 |
| 1.094 | 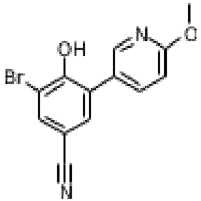 | | 1.90 | 303.0 |
| 1.095 | 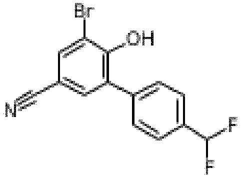 | | 2.27 | 324.0 |
| 1.096 | 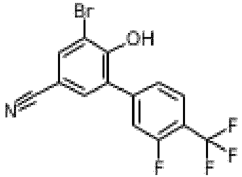 | | 2.47 | 359.98 |
| 1.097 | 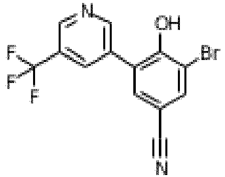 | | 2.06 | 343.0 |
| 1.098 | 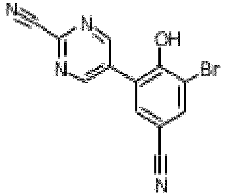 | | 1.82 | 300.1 |
| 1.099 | 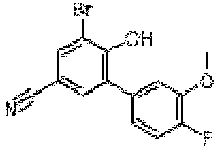 | | 2.24 | 320.0 |
| 1.100 | 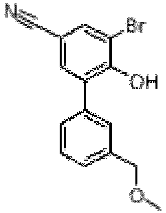 | | 2.17 | 316.1 |
| 1.101 | 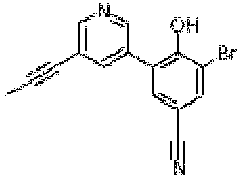 | | 1.76 | 313.0 |
| 1.102 | 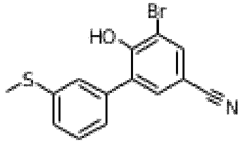 | | 2.37 | 320.9 |
| 1.103 | 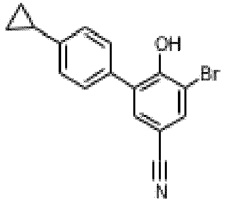 | | 2.51 | 314.0 |
| 1.104 | 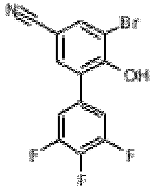 | | 2.35 | 327.9 |
| 1.105 | 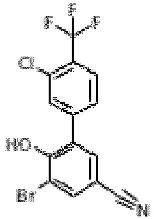 | | 2.57 | 374.0 |
| 1.106 | 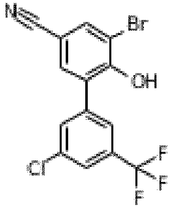 | | 2.60 | 374.0 |
| 1.107 | 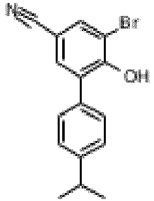 | | 2.63 | 316.0 |
| 1.108 | 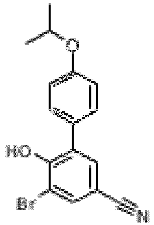 | | 2.49 | 332.0 |
| 1.109 | 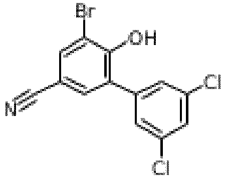 | | 2.57 | 339.9 |
| 1.110 | 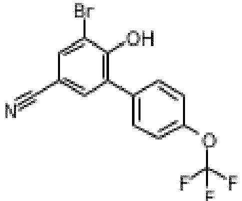 | | 2.49 | 356.0 |
| 1.111 | 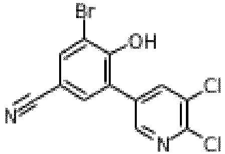 | | 2.26 | 340.9 |
| 1.112 | 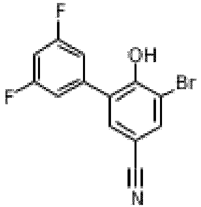 | | 2.29 | 310.0 |
| 1.113 | 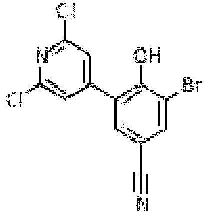 | | 2.28 | 341.0 |
| 1.114 | 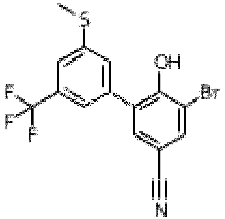 | | 2.60 | 388.0 |
| 1.115 | 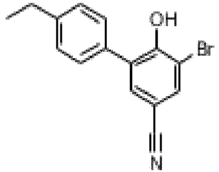 | | 2.51 | 303.9 |
| 1.116 | 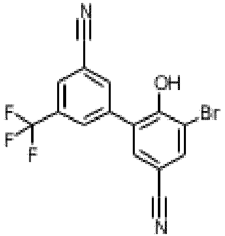 | | 2.34 | 365.1 |
| 1.117 | 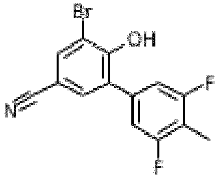 | | 2.45 | 324.0 |
| 1.118 | 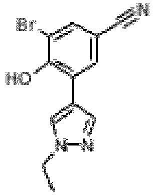 | | 1.80 | 292.0 |
| 1.119 | 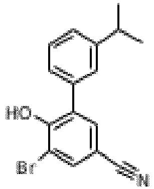 | | 2.63 | 316.1 |
| 1.120 | 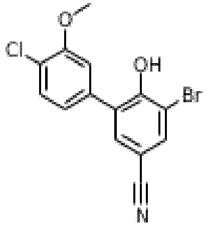 | | 2.38 | 338.0 |
| 1.121 | 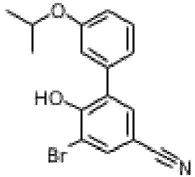 | | 2.49 | 332.0 |
| 1.122 | 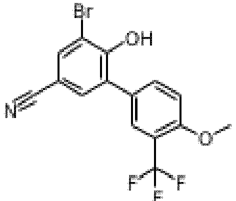 | | 2.44 | 370.1 |
| 1.123 | 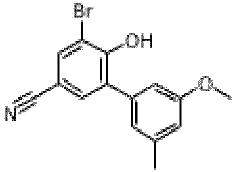 | | 2.38 | 318.0 |
| 1.124 | 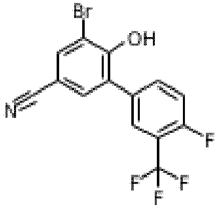 | | 2.45 | 358.0 |
| 1.125 | 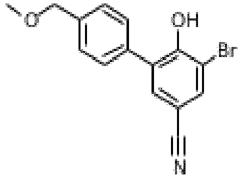 | | 2.19 | 318.0 |
| 1.126 | 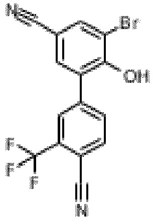 | | 2.31 | 367.0 |
| 1.127 | 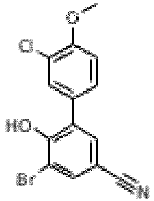 | | 2.35 | 337.9 |
| 1.128 | 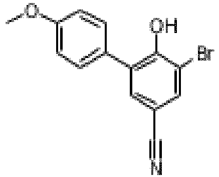 | | 2.23 | 303.9 |
| 1.129 | 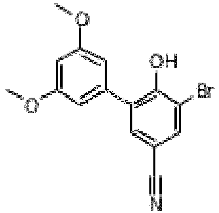 | | 2.27 | 334.0 |
| 1.130 | 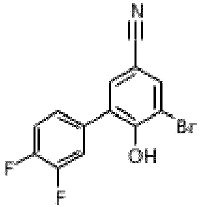 | | 2.29 | 309.9 |
| 1.131 | 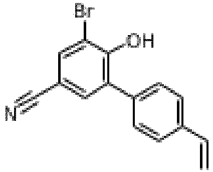 | | 2.43 | 299.9 |
| 1.132 | 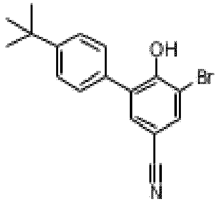 | | 2.73 | 330.2 |
| 1.133 | 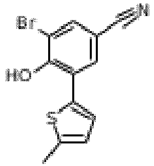 | | 2.33 | 293.9 |
| 1.134 | 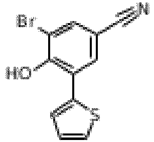 | | 2.15 | 279.9 |
| 1.135 | 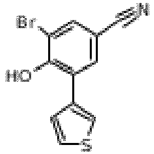 | | 2.15 | 280.2 |
| 1.136 | 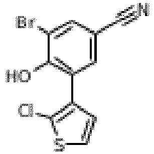 | | 2.26 | 313.9 |
| 1.137 | 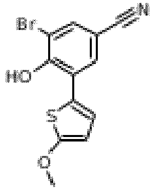 | | 2.24 | 309.9 |
| 1.138 | 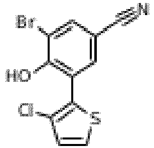 | | 2.36 | 313.9 |
| 1.139 | 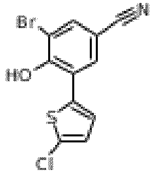 | | 2.41 | 313.9 |
| 1.140 | 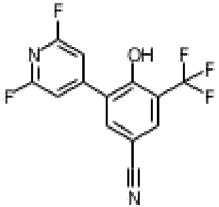 | | 2.17 | 299.1 |
| 1.141 | 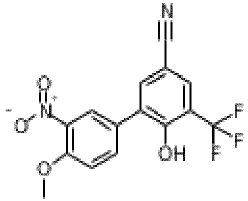 | | 2.23 | 337.1 |
| 1.142 | 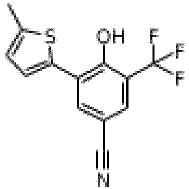 | | 2.36 | 282.1 |
| 1.143 | 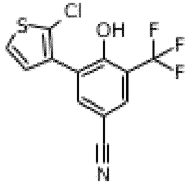 | | 2.31 | 302.0 |
| 1.144 | 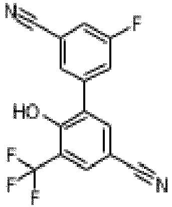 | | 2.21 | 305.1 |
| 1.145 | 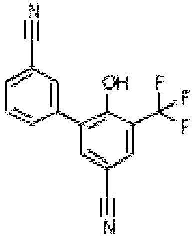 | | 2.14 | 287.1 |
| 1.146 | 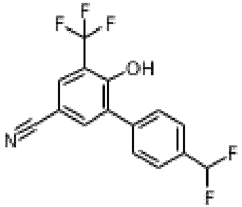 | | 2.34 | 312.1 |
| 1.147 | 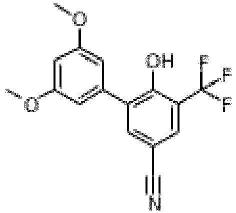 | | 2.35 | 322.2 |
| 1.148 | 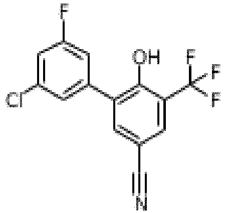 | | 2.45 | 314.1 |
| 1.149 | 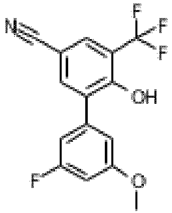 | | 2.36 | 310.2 |
| 1.150 | 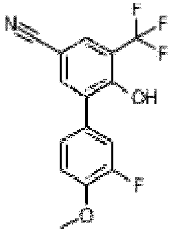 | | 2.30 | 310.1 |
| 1.151 | 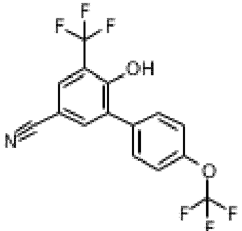 | | 2.53 | 346.1 |
| 1.152 | 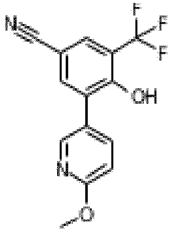 | | 2.05 | 293.2 |
| 1.153 | 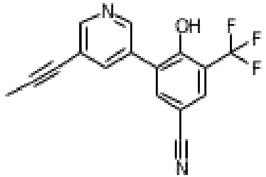 | | 1.92 | 301.1 |
| 1.154 | 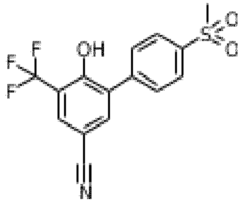 | | 1.96 | 340.1 |
| 1.155 | 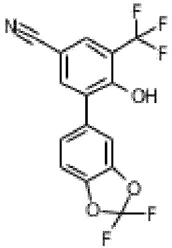 | | 2.48 | 342.1 |
| 1.156 | 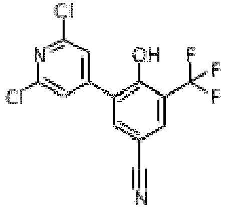 | | 2.34 | 331.1 |
| 1.157 | 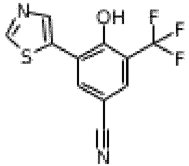 | (CDCL₃) δ = 8.11 (d, 1 H) 7.92 (d, H) 7.89 (d, 1H) 7.50 (d, 1H) | 1.61 | 269.0 |
| 1.158 | 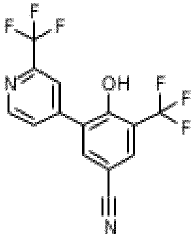 | | 2.20 | 331.1 |
| 1.159 | 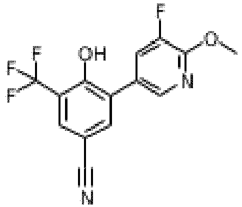 | | 2.21 | 311.1 |
| 1.160 | 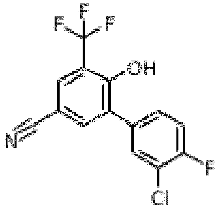 | | 2.43 | 314.1 |
| 1.161 | 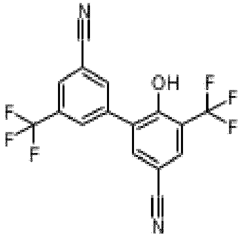 | | 2.38 | 355.2 |
| 1.162 | 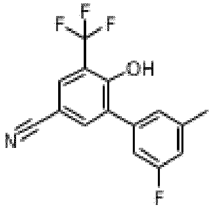 | | 2.43 | 294.1 |
| 1.163 | 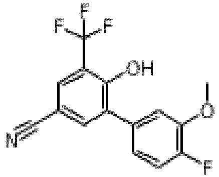 | | 2.31 | 310.1 |
| 1.164 | 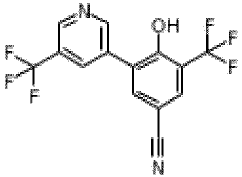 | | 2.15 | 331.1 |
| 1.165 | 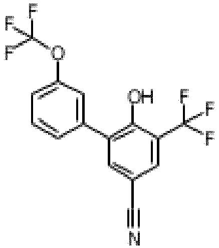 | | 2.51 | 346.2 |
| 1.166 | 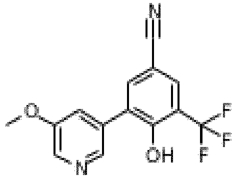 | | 1.50 | 293.1 |
| 1.167 | 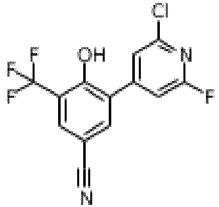 | | 2.26 | 315.1 |
| 1.168 | 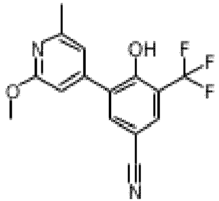 | | 1.88 | 307.1 |
| 1.169 | 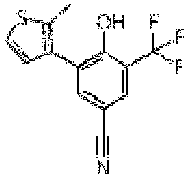 | | 2.33 | 282.1 |
| 1.170 | 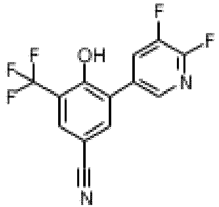 | | 2.12 | 299.1 |
| 1.171 | 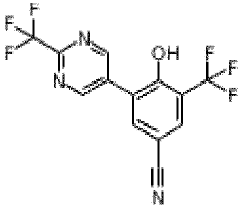 | | 2.13 | 332.1 |
| 1.172 | 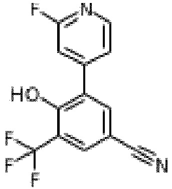 | | 1.98 | 281.1 |
| 1.173 | 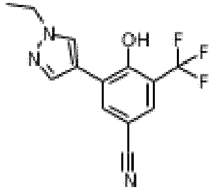 | | 1.91 | 280.1 |
| 1.174 | 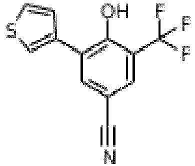 | | 2.20 | 268.1 |
| 1.175 | 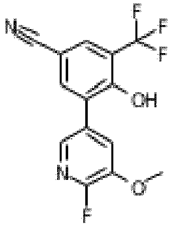 | | 2.08 | 311.1 |
| 1.176 | 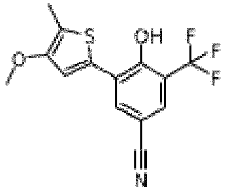 | | 2.41 | 312.1 |
| 1.177 | 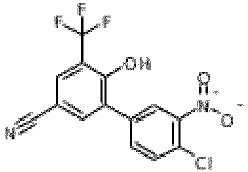 | | 2.35 | 341.1 |
| 1.178 | 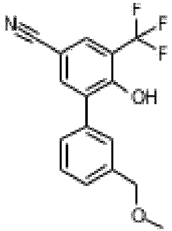 | | 2.27 | 306.1 |
| 1.179 | 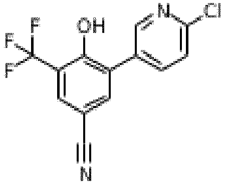 | | 2.07 | 297.1 |
| 1.180 | 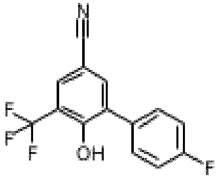 | | 2.29 | 280.1 |
| 1.181 | 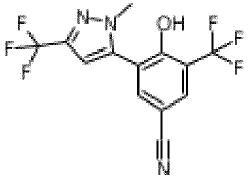 | | 2.24 | 334.2 |
| 1.182 | 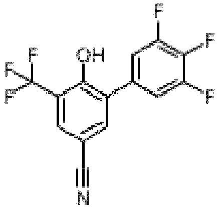 | (CDCL₃) δ = 7.92-7.86 (m, 1H), 7.69 (s, 1H), 7.17 - 7.11 (m, 2H) | | |
| 1.183 | 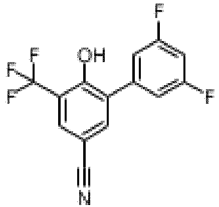 | (CDCL₃) δ = 7.91 (d, 1H), 7.72 (d, 1H), 7.05 - 6.94 (m, 3H) | | |
| 1.184 | 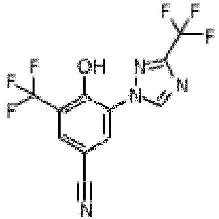 | (CDCL₃) δ = 10.52 (bs, 1H), 8.89 (s, 1H), 8.05 (d, 1H), 7.98 (d, 1H) | | |
| 1.185 | 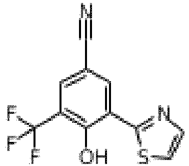 | (CDCL₃) δ = 8.11 (d, 1 H) 7.92 (d, H) 7.89 (d, 1H) 7.50 (d, 1H) | | |
| 1.186 | 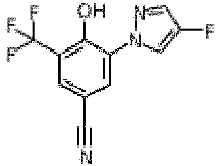 | (CDCL₃) δ = 7.69-7.74 (m, 2 H) 7.79 (d, 1H) 7.96 (d, 1H) 12.79 (s, 1H) . | | |
| 1.187 | 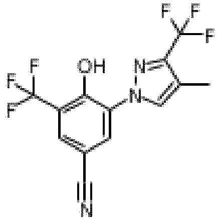 | (CDCL₃) δ = 12.24 (s, 1 H) 7.94 (s, 1 H) 7.84 (s, 2 H) 2.32 (s, 3H) | | |
| 1.188 | 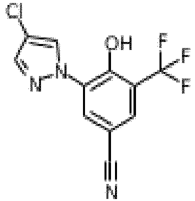 | (CDCL₃) δ = 12.68 (m, 1H), 807 (s, 1H), 7.82 - 7.80 (m, 1H), 7.78 - 7.77 (m, 2H) | | |
| 1.189 | 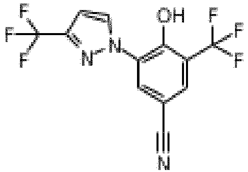 | (CDCL₃) δ = 12.02 (bs, 1H), 8.16 (d,1H), 7.92 (d, 1H), 7.88 (d, 1H), 6.94 (d, 1H). | | |
| 1.190 | 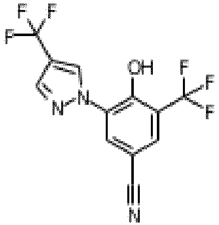 | (CDCL₃) δ = 12.32 (s, 1H) 8.40 (s, 1H), 8.06 (s, 1 H), 7.90 (d, 1H) 7.87 (d, 1H) | | |
| 1.191 | 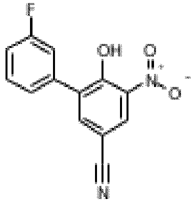 | (DMSO-d6) δ = 8.53 (d, 1H) 8.11 (d, 1 H), 7.53 (m, 1H), 7.40 (m, 2H) 7.28 (m, 1H) | | |
| 1.192 | 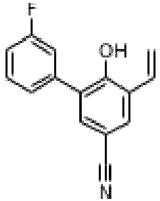 | (DMSO-d6) δ = 10.02 (s, 1H), 7.97-8.00 (d, *J* =*8.0* HZ, 1H), 7.63-7.64 (d, *J* = *4.0* HZ, 1H),7.46-7.52 (q, *J* = *4.0* HZ,1 H), 7.34-7.37 (m, *J* = *4.0* HZ, 2H), 7.20-7.25(t, *J* = *4.0* HZ ,1H), 7.02-7.09 (dd, *J* = *12.0* HZ, 1H), 5.94-5.99 (d, *J* = *4.0* HZ, 1H), 5.40-5.43 (d, *J* = *4.0* HZ, 1H). | | |
| 1.193 | 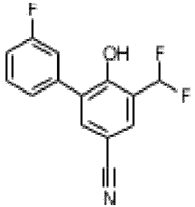 | (DMSO-d6) δ = 10.66 (s, 1H), 7.93 (d, 1H), 7.87 (d, 1H), 7.49-7.55 (m, 1H), 7.33-7.38 (m, 2H), 7.05-7.28(m, 2H) | | |
| 1.194 | 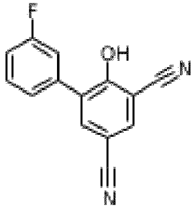 | (DMSO-d6) δ = 8.30 (d, 1H), 8.01 (d, 1H), 7.46-7.55 (q, *J* = *8.0* HZ, 1H), 7.36-7.42 (q, *J* = *8.0* HZ, 2H), 7.21-7.29(t, *J* = *8.0* HZ, 1H | | |
| 1.195 | 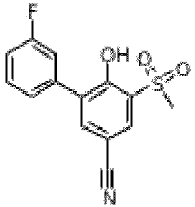 | (DMSO-d6) δ = 8.10-8.11 (d, *J* = *4.0* HZ, 1H), 800 (d, 1H), 7.50-7.55 (q, *J* = *4.0* HZ, 1H), 7.36-7.43(q, *J* = *8.0* HZ, 2H), 7.24-7.29 (t, *J* = *4.0* HZ, 1H), 3.35 (s, 3H) | | |
| 1.196 | 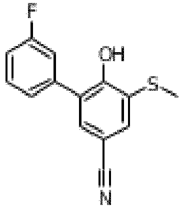 | (DMSO-d6) δ = 10.02-9.98 (s, 1H), 7.60-7.59 (d, *J* = *4.0* HZ, 1H) 7.54 (d, 1H), 7.46-7.52 (q, *J* = *8.0* HZ, 1H), 7.33-7.36 (t, *J* = *4.0* HZ, 2H), 7.20-7.25(t, *J* = *8.0* HZ, 1H), 2.47 (s, 3H) | | |
| 1.197 | 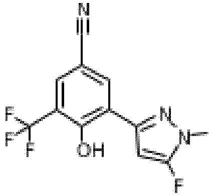 | | 3.25 | 285.2 |
| 1.198 | 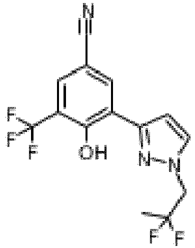 | | | |
| 1.199 | 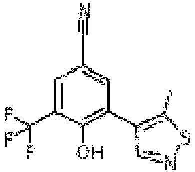 | | 2.62 | 284.3 |
| 1.200 | 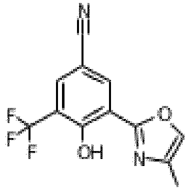 | | | |
| 1.201 | 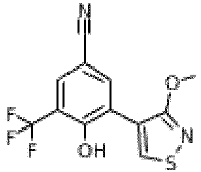 | | 2.73 | 300.3 |
| 1.202 | 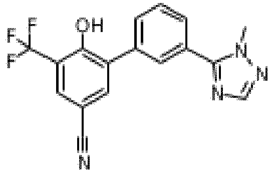 | | | |
| 1.203 | 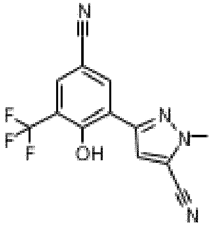 | | | |
| 1.204 | 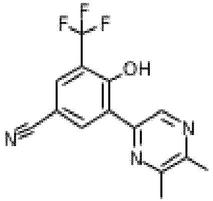 | | | |
| 1.205 | 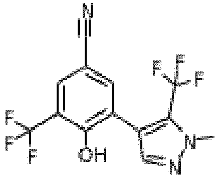 | | 2.83 | 335.2 |
| 1.206 | 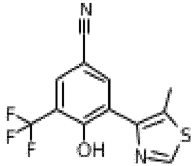 | | 3.15 | 284.3 |
| 1.207 | 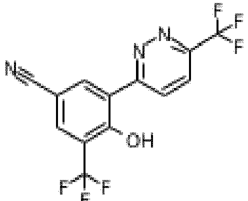 | | 3.29 | 333.2 |
| 1.208 | 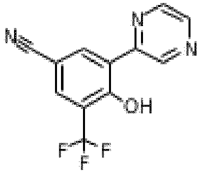 | | 2.73 | 265.2 |
| 1.209 | 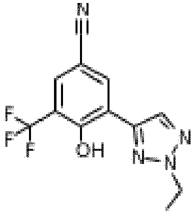 | | 3.19 | 282.2 |
| 1.210 | 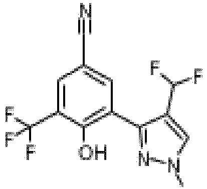 | | | |
| 1.211 | 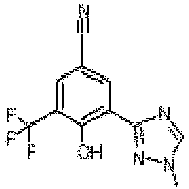 | | | |
| 1.212 | 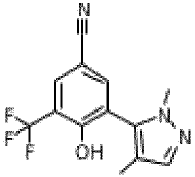 | | | |
| 1.213 | 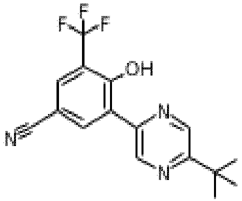 | | | |
| 1.214 | 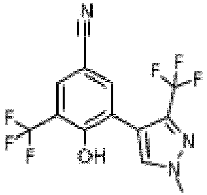 | | 2.81 | 335.2 |
| 1.215 | 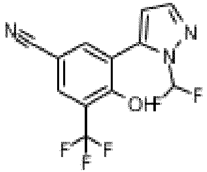 | | | |
| 1.216 | 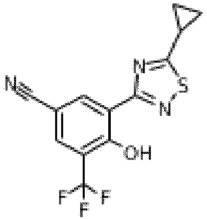 | | | |
| 1.217 | 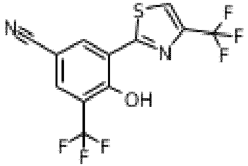 | | 3.49 | 338.2 |
| 1.218 | 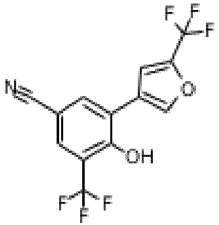 | | | |
| 1.219 | 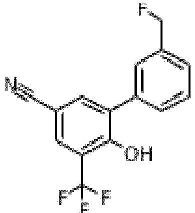 | | | |
| 1.220 | 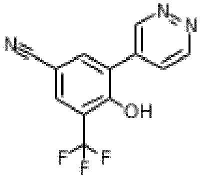 | | | |
| 1.221 | 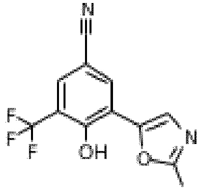 | | | |
| 1.222 | 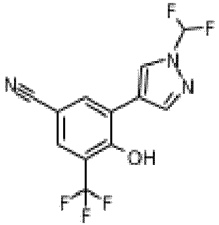 | | 2.60 | 303.2 |
| 1.223 | 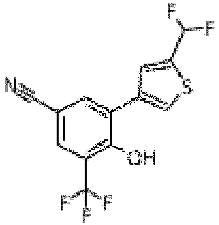 | | 3.06 | 319.2 |
| 1.224 | 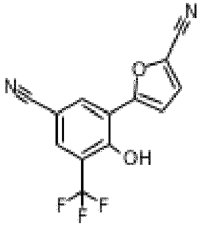 | | | |
| 1.225 | 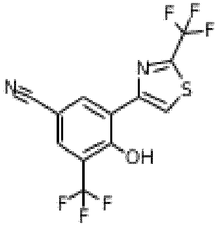 | | 3.60 | 338.2 |
| 1.226 | 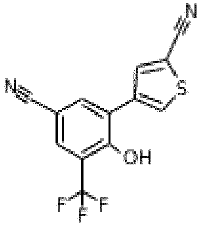 | | 2.79 | 294.3 |
| 1.227 | 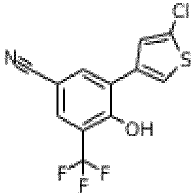 | | 3.21 | 303.7 |
| 1.228 | 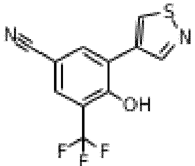 | | 2.44 | 270.2 |
| 1.229 | 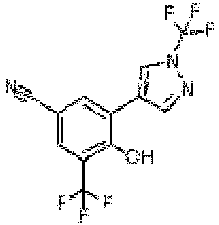 | | 2.87 | 321.2 |
| 1.230 | 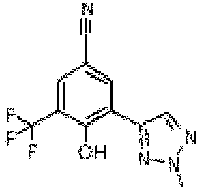 | | 2.92 | 268.2 |
| 1.231 | 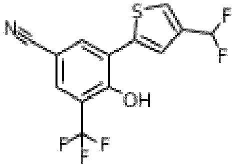 | | | |
| 1.232 | 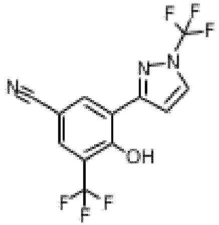 | | 3.48 | 321.2 |
| 1.233 | 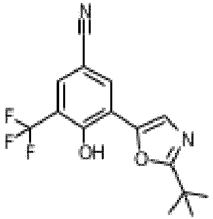 | | | |
| 1.234 | 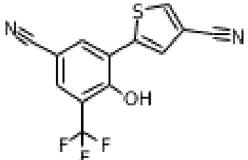 | | 2.74 | 294.3 |
| 1.235 | 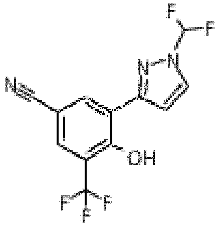 | | 3.21 | 303.2 |
| 1.236 | 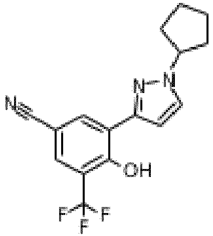 | | | |
| 1.237 | 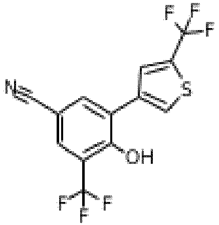 | | 3.31 | 337.2 |
| 1.238 | 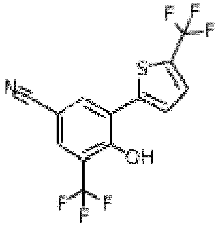 | | | |
| 1.239 | 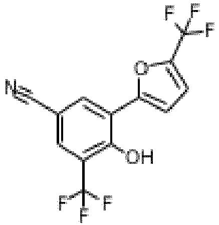 | | | |
| 1.240 | 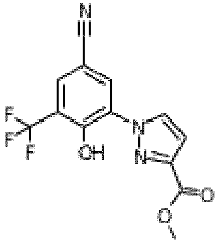 | | | |
| 1.241 | 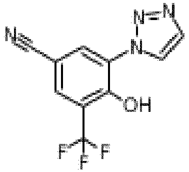 | | 2.08 | 254.2 |
| 1.242 | 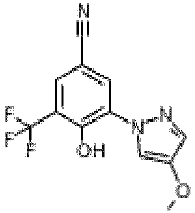 | | | |
| 1.243 | 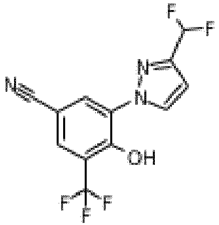 | | 3.15 | 303.2 |
| 1.244 | 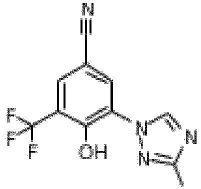 | | 2.37 | 268.2 |
| 1.245 | 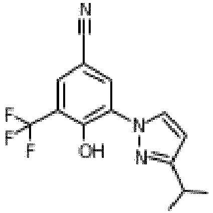 | | 3.78 | 295.3 |
| 1.246 | 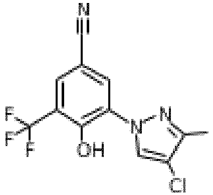 | | 3.60 | 301.7 |
| 1.247 | 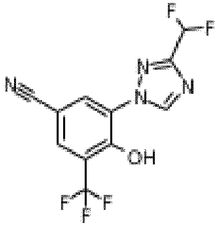 | | 2.35 | 304.2 |
| 1.248 | 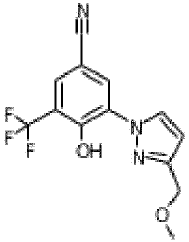 | | 3.06 | 297.2 |
| 1.249 | 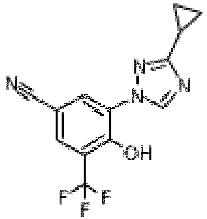 | | 2.85 | 294.2 |
| 1.250 | 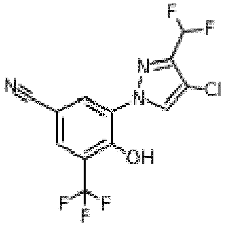 | | 3.26 | 337.6 |
| 1.251 | 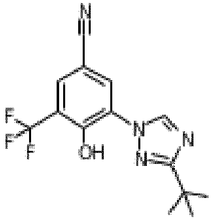 | | | |
| 1.252 | 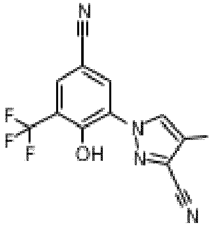 | | 3.04 | 292.2 |
| 1.253 | 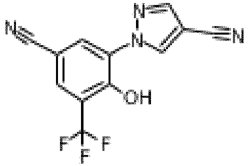 | | | |
| 1.254 | 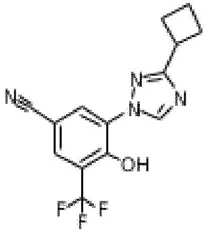 | | 3.12 | 308.3 |
| 1.255 | 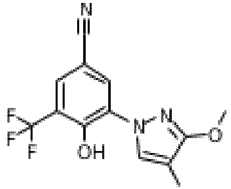 | | | |
| 1.256 | 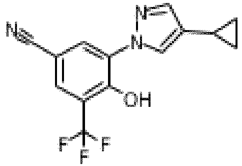 | | | |
| 1.257 | 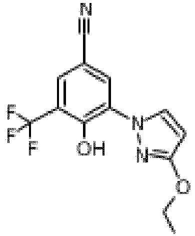 | | 3.45 | 297.2 |
| 1.258 | 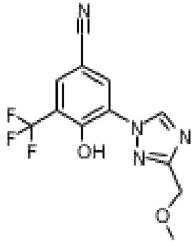 | | 2.21 | 298.2 |
| 1.259 | 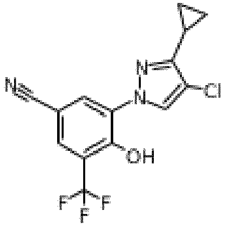 | | 3.85 | 327.7 |
| 1.260 | 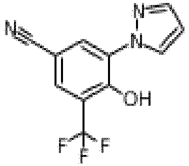 | | 3.06 | 253.2 |
| 1.261 | 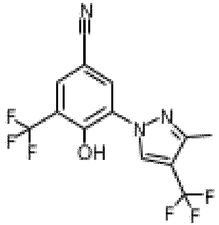 | | 3.63 | 335.2 |
| 1.262 | 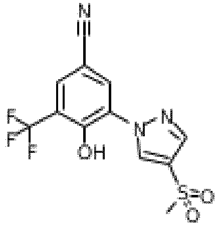 | | | |
| 1.263 | 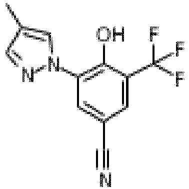 | | 3.33 | 297.2 |
| 1.264 | 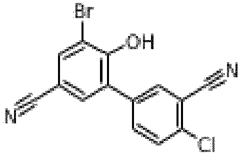 | | 2.25 | 331.0 |
| 1.265 | 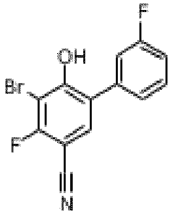 | δ = 7.56 - 7.43 (m, 2H), 7.28 - 7.12 (m, 3H), 6.34 (s, 1H) | | |
| 1.266 | 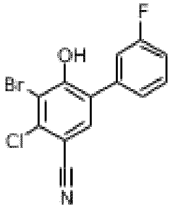 | δ = 7.61 (s, 1H), 7.45 (m, 1H), 7.26-7.24 (m, 2H), 7.14 (m, 1H), 6.36 (s, 1H) | | |
| 1.267 | 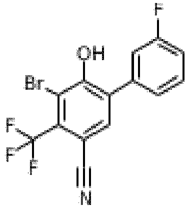 | δ =7.75 (s, 1H), 7.48 (m, 1H), 730 (m, 2H), 7.18 (m, 1H), 6.74 (s, 1H) | | |
| 1.268 | 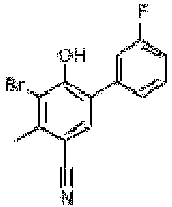 | δ = 7.55 (s, 1H), 7.43 (m, 1H), 7.27-7.25 (m, 2H), 7.11 (m, 1H), 6.28 (s, 1H), 2.68 (s, 3H) | | |

**Table 2 - Examples of herbicidal compounds of the present invention.**

| **Compound** | **Structure** | **¹HNMR** |
|---|---|---|
| 2.001 | 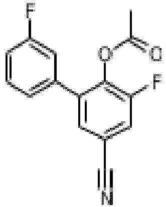 | (DMSO-d6) δ 8.15- 8.13 (d, 1H), 7.95 (s, 1H), 7.58-7.53 (q, 1H), 7.35-7.29 (m, 3H), 2.24 (s, 3H) |
| 2.002 | 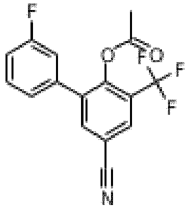 | (DMSO-d₆) δ 8.46 (s, 1H), 8.39 (s, 1H), 7.58-7.52 (q, 1H), 7.40-7.30 (m, 3H), 2.10 (s, 3H). |
| 2.003 | 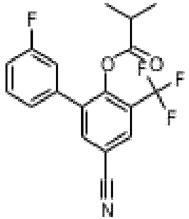 | (DMSO-d₆) δ 8.47 (s, 1H), 8.38 (s, 1H), 7.57-7.52 (q, 1H), 7.35-7.29 (m, 3H), 2.66-2.50 (m, 1H), 0.96 (brs, 6H). |
| 2.004 | 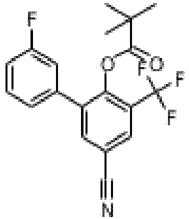 | (DMSO-d₆) δ 8.47 (s, 1H), 8.35 (d, 1H), 7.57-7.52 (q, 1H), 7.34-7.25 (m, 3H), 1.02 (s, 9H). |
| 2.005 | 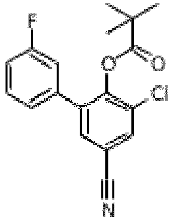 | (DMSO-d₆) δ 8.30 (d, 1H), 8.02 (d, 1H), 7.56-7.50 (q, 1H), 7.33-7.23 (m, 3H), 1.12 (s, 9H). |
| 2.006 | 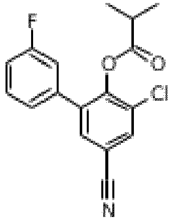 | (DMSO-d₆)δ 8.30 (d, 1H), 8.03 (d, 1H), 7.56-7.51 (q, 1H), 7.33-7.25 (m, 3H), 2.77-2.67 (m, 1H), 1.06-1.04 (d, 6H) |

### Biological Examples

Seeds of a variety of test species are sown in standard soil in pots (*Ipomoea hederacea (IPOHE), Abutilon theophrasti (ABUTH), Amaranthus retoflexus* (AMARE), *Echinochloa crus-galli* (ECHCG), *Setaria faberi* (SETFA)). After 8 days cultivation under controlled conditions in a glasshouse (at 24/16°C, day/night; 14 hours light; 65 % humidity), the plants are sprayed with an aqueous spray solution derived from the formulation of the technical active ingredient in acetone / water (50:50) solution containing 0.5% Tween 20 (polyoxyethelyene sorbitan monolaurate, CAS RN 9005-64-5). Compounds are applied at 1000 g/ha unless otherwise stated. The test plants are then grown in a glasshouse under controlled conditions in a glasshouse (at 24/16°C, day/night; 14 hours light; 65 % humidity) and watered twice daily. After 13 days the test is evaluated for the percentage damage caused to the plant. The biological activities are shown in the following table on a five-point scale (5 = 81-100%; 4 = 61-80%; 3=41-60%; 2=21-40%; 1=0-20%).

**TABLE B1 Post-emergence Test**

| **Compound** | **IPOHE** | **ABUTH** | **AMARE** | **ECHCG** | **SETFA** |
|---|---|---|---|---|---|
| 1.001 | 1 | 5 | 4 | - | 4 |
| 1.002 | 5 | 4 | 5 | 3 | 2 |
| 1.004 | 1 | 5 | 5 | 3 | 3 |
| 1.005 | 5 | 5 | 4 | 4 | 1 |
| 1.007 | 3 | 2 | 5 | - | 1 |
| 1.008 | 2 | 2 | 5 | - | 4 |
| 1.009 | 2 | 2 | 4 | - | 2 |
| 1.027 | 3 | 4 | 5 | - | 4 |
| 1.032 | 3 | 4 | 5 | - | 4 |
| 1.034 | - | 4 | 4 | - | - |
| 1.036 | 3 | 2 | 5 | 3 | 4 |
| 1.044 | 1 | 5 | 5 | 2 | 3 |
| 1.045 | 1 | 3 | 5 | 1 | 1 |
| 1.046 | 3 | 5 | 5 | 2 | 3 |
| 1.047 | 3 | 5 | 5 | 1 | 2 |
| 1.048 | 2 | 5 | 5 | 2 | 2 |
| 1.052 | 1 | 5 | 5 | 1 | 3 |
| 1.053 | 3 | 2 | 5 | 1 | 1 |
| 1.054 | 2 | 5 | 5 | 3 | 2 |
| 1.058 | 2 | 1 | 5 | 2 | 3 |
| 1.060 | 2 | 4 | 5 | 1 | 1 |
| 1.061 | 3 | 3 | 5 | 1 | 1 |
| 1.067 | 2 | 3 | 1 | 2 | 2 |
| 1.130 | 2 | 5 | 5 | 1 | 1 |
| 1.134 | 4 | 4 | 4 | 1 | 1 |
| 1.135 | 5 | 5 | 2 | 1 | 1 |
| 1.145* | 3 | 1 | 1 | 1 | 1 |
| 1.148* | 4 | 1 | 3 | 1 | 1 |
| 1.151 | 3 | 1 | 5 | 1 | 1 |
| 1.158 | 2 | 1 | 4 | 1 | 1 |
| 1.160 | 2 | 1 | 5 | 1 | 1 |
| 1.162 | 3 | 1 | 2 | 1 | 1 |
| 1.165 | 4 | 1 | 3 | 1 | 1 |
| 1.180 | 3 | 3 | 1 | 1 | 1 |
| 1.182 | 5 | 4 | 5 | 1 | 1 |
| 1.183 | 2 | 4 | 5 | 2 | 2 |
| 1.186 | 4 | 5 | 2 | 1 | 1 |
| 1.187 | 1 | 1 | 5 | 1 | 1 |
| 1.190 | 1 | 1 | 3 | 1 | 1 |
| 1.191 | 3 | 4 | 5 | 2 | 2 |
| 1.194 | 3 | 3 | 2 | 1 | 2 |
| 1.225 | - | - | 5 | - | - |
| 1.250 | 5 | 2 | 5 | 2 | 2 |
| 1.259 | 5 | 3 | 5 | 2 | 2 |
| 2.002 | 5 | 5 | 5 | 4 | 1 |
| 2.006 | 1 | 4 | 3 | 1 | 1 |

| | | | | | |
|---|---|---|---|---|---|
| *Applied at 500gai/ha | | | | | |

## Claims

1. A compound of Formula (I): 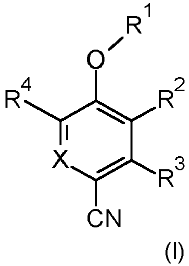 or an agronomically acceptable salt thereof,
wherein
X is CH or N,
R¹ is selected from the group consisting of hydrogen, C₁-C₁₀alkylC(O)-, C₁-C₁₀alkylOC(O)-, C₁-C₁₀alkylSC(O)- and R⁵R⁶NC(O)-;
R² is selected from the group consisting of halogen NO₂, CN, C₁-C₆haloalkylS(O)ₚ-, C₁-C₆alkyl-S(O)ₚ-, C₂-C₄alkenyl-, C₂-C₄alkynyl-, C₁-C₄haloalkyl and C₁-C₄haloalkoxy-;
R³ is selected from the group consisting of hydrogen, halogen, C₁-C₄alkyl, C₁-C₄haloalkyl, C₁-C₄alkoxy- and C₁-C₄haloalkoxy-;
R⁴ is phenyl or a five or six-membered heteroaryl, the heteroaryl containing from one to three heteroatoms each independently selected from the group consisting of oxygen, nitrogen and sulphur, and wherein the phenyl or heteroaryl may be optionally substituted by one or more substituents independently selected from the group consisting of halogen, C₁-C₆alkyl-, C₂-C₄alkenyl-, C₂-C₄alkynyl-, C₁-C₆haloalkyl-, C₁-C₆alkoxy-, C₁-C₆alkoxyC₁-C₃alkyl-, C₁-C₆alkoxyC₁-C₃alkoxy-, C₁-C₆haloalkoxy-, C₁-C₆alkyl-S(O)ₚ-, C₁-C₆haloalkyl-S(O)ₚ-, C₃-C₆cycloalkyl- (optionally substituted by 1 or 2 halogen), cyano and nitro; or
R⁴ is R4^{a} 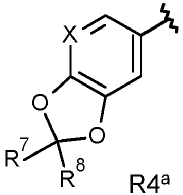 and
R⁵ is hydrogen or C₁-C₁₀alkyl;
R⁶ is hydrogen or C₁-C₁₀alkyl;
R⁷ and R⁸ are independently selected from the group consisting of hydrogen and halogen; and
p = 0, 1 or 2;
wherein said compound is not selected from the group consisting of 2-(3-bromo-5-cyano-2-hydroxyphenyl)pyridine, 3-chloro-2-ethoxy-4-hydroxy-5-phenyl-benzonitrile, 4-hydroxy-3-nitro-5-[3-(trifluoromethyl)phenyl]benzonitrile and 2'-fluoro-6-hydroxy-5-nitrobiphenyl-3-carbonitrile.

2. A compound according to claim 1, wherein R¹ is hydrogen.

3. A compound according to claim 1 or claim 2, wherein R² is selected from the group consisting of F, Cl, Br, -CF₃ and -OCF₃.

4. A compound according to claim 3, wherein R² is CF₃.

5. A compound according to any one of the previous claims, wherein R³ is hydrogen.

6. A compound according to any one of claims 1 to 5, wherein R⁴ is phenyl.

7. A compound according to any one of claims 1 to 5, wherein R⁴ is an optionally substituted five or six-membered heteroaryl selected from the group consisting of furanyl, thiophenyl, thiazolyl, oxazolyl, isoxazolyl, pyrazolyl, isothiazolyl, pyridyl, pyridazinyl, pyrazinyl, pyrimidinyl and triazolyl.

8. A compound according to claim 7, wherein R⁴ is an optionally substituted five or six-membered heteroaryl selected from the group consisting of pyrazolyl, thiazolyl and thiophenyl.

9. A compound according to claim 8, wherein R⁴ is pyridyl or pyrazolyl.

10. A compound according to any one of the previous claims, wherein X is CH.

11. A herbicidal composition comprising a compound according to any one of the previous claims and an agriculturally acceptable formulation adjuvant.

12. A herbicidal composition according to claim 11, further comprising at least one additional pesticide.

13. A herbicidal composition according to claim 12, wherein the additional pesticide is a herbicide or herbicide safener.

14. A method of controlling weeds at a locus comprising application to the locus a composition comprising a weed controlling amount of a compound of Formula (I): 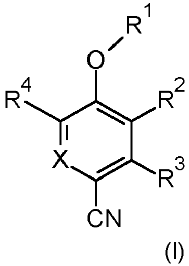 or an agronomically acceptable salt thereof,
wherein
X is CH or N,
R¹ is selected from the group consisting of hydrogen, C₁-C₁₀alkylC(O)-, C₁-C₁₀alkylOC(O)-, C₁-C₁₀alkylSC(O)- and R⁵R⁶NC(O)-;
R² is selected from the group consisting of halogen NO₂, CN, C₁-C₆haloalkylS(O)ₚ-, C₁-C₆alkyl-S(O)ₚ-, C₂-C₄alkenyl-, C₂-C₄alkynyl-, C₁-C₄haloalkyl and C₁-C₄haloalkoxy-;
R³ is selected from the group consisting of hydrogen, halogen, C₁-C₄alkyl, C₁-C₄haloalkyl, C₁-C₄alkoxy- and C₁-C₄haloalkoxy-;
R⁴ is phenyl or a five or six-membered heteroaryl, the heteroaryl containing from one to three heteroatoms each independently selected from the group consisting of oxygen, nitrogen and sulphur, and wherein the phenyl or heteroaryl may be optionally substituted by one or more substituents selected from the group consisting of halogen, C₁-C₆alkyl-, C₂-C₄alkenyl-, C₂-C₄alkynyl-, C₁-C₆haloalkyl-, C₁-C₆alkoxy-, C₁-C₆alkoxyC₁-C₃alkyl-, C₁-C₆alkoxyC₁-C₃alkoxy-, C₁-C₆haloalkoxy-, C₁-C₆alkyl-S(O)ₚ-, C₁-C₆haloalkyl-S(O)ₚ-, C₃-C₆cycloalkyl- (optionally substituted by 1 or 2 halogen), cyano and nitro; or
R⁴ is R4^{a} 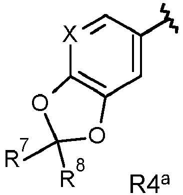 and
R⁵ is hydrogen or C₁-C₁₀alkyl;
R⁶ is hydrogen or C₁-C₁₀alkyl;
R⁷ and R⁸ are independently selected from the group consisting of hydrogen and halogen; and
p = 0, 1 or 2.

15. Use of a compound of a compound of Formula (I): 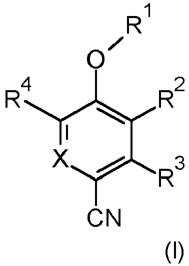 or an agronomically acceptable salt thereof,
wherein
X is CH or N,
R¹ is selected from the group consisting of hydrogen, C₁-C₁₀alkylC(O)-, C₁-C₁₀alkylOC(O)-, C₁-C₁₀alkylSC(O)- and R⁵R⁶NC(O)-;
R² is selected from the group consisting of halogen NO₂, CN, C₁-C₆haloalkylS(O)ₚ-, C₁-C₆alkyl-S(O)ₚ-, C₂-C₄alkenyl-, C₂-C₄alkynyl-, C₁-C₄haloalkyl and C₁-C₄haloalkoxy-;
R³ is selected from the group consisting of hydrogen, halogen, C₁-C₄alkyl, C₁-C₄haloalkyl, C₁-C₄alkoxy- and C₁-C₄haloalkoxy-;
R⁴ is phenyl or a five or six-membered heteroaryl, the heteroaryl containing from one to three heteroatoms each independently selected from the group consisting of oxygen, nitrogen and sulphur, and wherein the phenyl or heteroaryl may be optionally substituted by one or more substituents selected from the group consisting of halogen, C₁-C₆alkyl-, C₂-C₄alkenyl-, C₂-C₄alkynyl-, C₁-C₆haloalkyl-, C₁-C₆alkoxy-, C₁-C₆alkoxyC₁-C₃alkyl-, C₁-C₆alkoxyC₁-C₃alkoxy-, C₁-C₆haloalkoxy-, C₁-C₆alkyl-S(O)ₚ-, C₁-C₆haloalkyl-S(O)ₚ-, C₃-C₆cycloalkyl- (optionally substituted by 1 or 2 halogen), cyano and nitro; or
R⁴ is R4^{a} 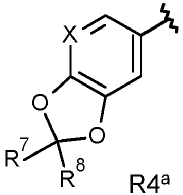 and
R⁵ is hydrogen or C₁-C₁₀alkyl;
R⁶ is hydrogen or C₁-C₁₀alkyl;
R⁷ and R⁸ are independently selected from the group consisting of hydrogen and halogen; and
p = 0, 1 or 2;
as a herbicide.

## Patentansprüche

1. Verbindung der Formel (I): 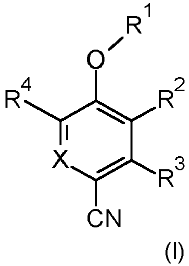 oder ein agronomisch unbedenkliches Salz davon,
wobei
X für C-H oder N steht,
R¹ aus der Gruppe bestehend aus Wasserstoff, C₁-C₁₀-Alkyl-C(O)-, C₁-C₁₀-Alkyl-OC(O)-, C₁-C₁₀-Alkyl-SC(O)- und R⁵R⁶NC(O)- ausgewählt ist;
R² aus der Gruppe bestehend aus Halogen, NO₂, CN, C₁-C₆-Halogenalkyl-S(O)ₚ-, C₁-C₆-Alkyl-S(O)ₚ-, C₂-C₄-Alkenyl-, C₂-C₄-Alkinyl-, C₁-C₄-Halogenalkyl und C₁-C₄-Halogenalkoxy- ausgewählt ist;
R³ aus der Gruppe bestehend aus Wasserstoff, Halogen, C₁-C₄-Alkyl, C₁-C₄-Halogenalkyl, C₁-C₄-Alkoxy- und C₁-C₄-Halogenalkoxy- ausgewählt ist;
R⁴ für Phenyl oder ein fünf- oder sechsgliedriges Heteroaryl steht, wobei das Heteroaryl ein bis drei Heteroatome, die jeweils unabhängig aus der Gruppe bestehend aus Sauerstoff, Stickstoff und Schwefel ausgewählt sind, enthält und wobei das Phenyl oder Heteroaryl gegebenenfalls durch einen oder mehrere Substituenten, die unabhängig aus der Gruppe bestehend aus Halogen, C₁-C₆-Alkyl-, C₂-C₄-Alkenyl-, C₂-C₄-Alkinyl-, C₁-C₆-Halogenalkyl-, C₁-C₆-Alkoxy-, C₁-C₆-Alkoxy-C₁-C₃-alkyl-, C₁-C₆-Alkoxy-C₁-C₃-alkoxy-, C₁-C₆-Halogenalkoxy-, C₁-C₆-Alkyl-S(O)ₚ-, C₁-C₆-Halogenalkyl-S(O)ₚ-, C₃-C₆-Cycloalkyl- (gegebenenfalls substituiert durch 1 oder 2 Halogen), Cyano und Nitro ausgewählt sind, substituiert sein kann; oder
R⁴ für R4^{a} steht 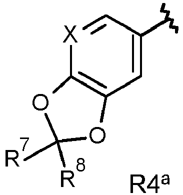 und
R⁵ für Wasserstoff oder C₁-C₁₀-Alkyl steht;
R⁶ für Wasserstoff oder C₁-C₁₀-Alkyl steht;
R⁷ und R⁸ unabhängig aus der Gruppe bestehend aus Wasserstoff und Halogen ausgewählt sind; und
p = 0, 1 oder 2;
wobei die Verbindung nicht aus der Gruppe bestehend aus 2-(3-Brom-5-cyano-2-hydroxyphenyl)pyridin, 3-Chlor-2-ethoxy-4-hydroxy-5-phenylbenzonitril, 4-Hydroxy-3-nitro-5-[3-(trifluormethyl)phenyl]benzonitril und 2'-Fluor-6-hydroxy-5-nitrobiphenyl-3-carbonitril ausgewählt ist.

2. Verbindung nach Anspruch 1, wobei R¹ für Wasserstoff steht.

3. Verbindung nach Anspruch 1 oder Anspruch 2, wobei R² aus der Gruppe bestehend aus F, Cl, Br, -CF₃ und -OCF₃ ausgewählt ist.

4. Verbindung nach Anspruch 3, wobei R² für CF₃ steht.

5. Verbindung nach einem der vorhergehenden Ansprüche, wobei R³ für Wasserstoff steht.

6. Verbindung nach einem der Ansprüche 1 bis 5, wobei R⁴ für Phenyl steht.

7. Verbindung nach einem der Ansprüche 1 bis 5, wobei R⁴ für ein gegebenenfalls substituiertes fünf- oder sechsgliedriges Heteroaryl steht, das aus der Gruppe bestehend aus Furanyl, Thiophenyl, Thiazolyl, Oxazolyl, Isoxazolyl, Pyrazolyl, Isothiazolyl, Pyridyl, Pyridazinyl, Pyrazinyl, Pyrimidinyl und Triazolyl ausgewählt ist.

8. Verbindung nach Anspruch 7, wobei R⁴ für ein gegebenenfalls substituiertes fünf- oder sechsgliedriges Heteroaryl steht, das aus der Gruppe bestehend aus Pyrazolyl, Thiazolyl und Thiophenyl ausgewählt ist.

9. Verbindung nach Anspruch 8, wobei R⁴ für Pyridyl oder Pyrazolyl steht.

10. Verbindung nach einem der vorhergehenden Ansprüche, wobei X für C-H steht.

11. Herbizide Zusammensetzung, umfassend eine Verbindung nach einem der vorhergehenden Ansprüche und ein landwirtschaftlich unbedenkliches Formulierungshilfsmittel.

12. Herbizide Zusammensetzung nach Anspruch 11, ferner umfassend mindestens ein zusätzliches Pestizid.

13. Herbizide Zusammensetzung nach Anspruch 12, wobei es sich bei dem zusätzlichen Pestizid um ein Herbizid oder einen Herbizid-Safener handelt.

14. Verfahren zur Bekämpfung von Unkräutern an einem Standort, bei dem man auf den Standort eine Zusammensetzung anwendet, die eine unkrautbekämpfende Menge einer Verbindung der Formel (I): 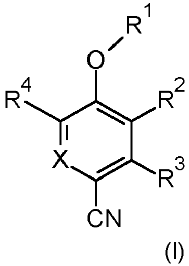 oder ein agronomisch unbedenkliches Salz davon umfasst, wobei
X für C-H oder N steht,
R¹ aus der Gruppe bestehend aus Wasserstoff, C₁-C₁₀-Alkyl-C(O)-, C₁-C₁₀-Alkyl-OC(O)-, C₁-C₁₀-Alkyl-SC(O)- und R⁵R⁶NC(O)- ausgewählt ist;
R² aus der Gruppe bestehend aus Halogen, NO₂, CN, C₁-C₆-Halogenalkyl-S(O)ₚ-, C₁-C₆-Alkyl-S(O)ₚ-, C₂-C₄-Alkenyl-, C₂-C₄-Alkinyl-, C₁-C₄-Halogenalkyl und C₁-C₄-Halogenalkoxy- ausgewählt ist;
R³ aus der Gruppe bestehend aus Wasserstoff, Halogen, C₁-C₄-Alkyl, C₁-C₄-Halogenalkyl, C₁-C₄-Alkoxy- und C₁-C₄-Halogenalkoxy- ausgewählt ist;
R⁴ für Phenyl oder ein fünf- oder sechsgliedriges Heteroaryl steht, wobei das Heteroaryl ein bis drei Heteroatome, die jeweils unabhängig aus der Gruppe bestehend aus Sauerstoff, Stickstoff und Schwefel ausgewählt sind, enthält und wobei das Phenyl oder Heteroaryl gegebenenfalls durch einen oder mehrere Substituenten, die aus der Gruppe bestehend aus Halogen, C₁-C₆-Alkyl-, C₂-C₄-Alkenyl-, C₂-C₄-Alkinyl-, C₁-C₆-Halogenalkyl-, C₁-C₆-Alkoxy-, C₁-C₆-Alkoxy-C₁-C₃-alkyl-, C₁-C₆-Alkoxy-C₁-C₃-alkoxy-, C₁-C₆-Halogenalkoxy-, C₁-C₆-Alkyl-S(O)ₚ-, C₁-C₆-Halogenalkyl-S(O)ₚ-, C₃-C₆-Cycloalkyl-(gegebenenfalls substituiert durch 1 oder 2 Halogen), Cyano und Nitro ausgewählt sind, substituiert sein kann; oder
R⁴ für R4^{a} steht 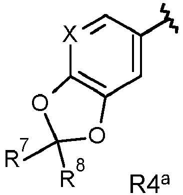 und
R⁵ für Wasserstoff oder C₁-C₁₀-Alkyl steht;
R⁶ für Wasserstoff oder C₁-C₁₀-Alkyl steht;
R⁷ und R⁸ unabhängig aus der Gruppe bestehend aus Wasserstoff und Halogen ausgewählt sind; und
p = 0, 1 oder 2.

15. Verwendung einer Verbindung der Formel (I): 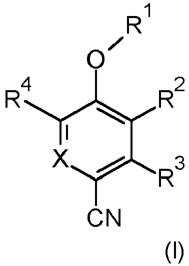 oder ein agronomisch unbedenkliches Salz davon,
wobei
X für C-H oder N steht,
R¹ aus der Gruppe bestehend aus Wasserstoff, C₁-C₁₀-Alkyl-C(O)-, C₁-C₁₀-Alkyl-OC(O)-, C₁-C₁₀-Alkyl-SC(O)- und R⁵R⁶NC(O)- ausgewählt ist;
R² aus der Gruppe bestehend aus Halogen, NO₂, CN, C₁-C₆-Halogenalkyl-S(O)ₚ-, C₁-C₆-Alkyl-S(O)ₚ-, C₂-C₄-Alkenyl-, C₂-C₄-Alkinyl-, C₁-C₄-Halogenalkyl und C₁-C₄-Halogenalkoxy- ausgewählt ist;
R³ aus der Gruppe bestehend aus Wasserstoff, Halogen, C₁-C₄-Alkyl, C₁-C₄-Halogenalkyl, C₁-C₄-Alkoxy- und C₁-C₄-Halogenalkoxy- ausgewählt ist;
R⁴ für Phenyl oder ein fünf- oder sechsgliedriges Heteroaryl steht, wobei das Heteroaryl ein bis drei Heteroatome, die jeweils unabhängig aus der Gruppe bestehend aus Sauerstoff, Stickstoff und Schwefel ausgewählt sind, enthält und wobei das Phenyl oder Heteroaryl gegebenenfalls durch einen oder mehrere Substituenten, die aus der Gruppe bestehend aus Halogen, C₁-C₆-Alkyl-, C₂-C₄-Alkenyl-, C₂-C₄-Alkinyl-, C₁-C₆-Halogenalkyl-, C₁-C₆-Alkoxy-, C₁-C₆-Alkoxy-C₁-C₃-alkyl-, C₁-C₆-Alkoxy-C₁-C₃-alkoxy-, C₁-C₆-Halogenalkoxy-, C₁-C₆-Alkyl-S(O)ₚ-, C₁-C₆-Halogenalkyl-S(O)ₚ-, C₃-C₆-Cycloalkyl-(gegebenenfalls substituiert durch 1 oder 2 Halogen), Cyano und Nitro ausgewählt sind, substituiert sein kann; oder
R⁴ für R4^{a} steht 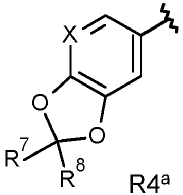 und
R⁵ für Wasserstoff oder C₁-C₁₀-Alkyl steht;
R⁶ für Wasserstoff oder C₁-C₁₀-Alkyl steht;
R⁷ und R⁸ unabhängig aus der Gruppe bestehend aus Wasserstoff und Halogen ausgewählt sind; und
p = 0, 1 oder 2;
als Herbizid.

## Revendications

1. Composé de formule (I) : 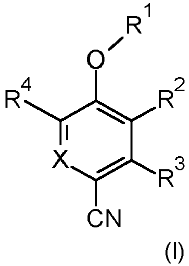 ou sel acceptable sur le plan agronomique correspondant,
X étant CH ou N,
R¹ étant choisi dans le groupe constitué par hydrogène, C₁-C₁₀alkylC(O)-, C₁-C₁₀alkylOC(O)-, C₁-C₁₀alkylSC(O)- et R⁵R⁶NC(O)- ;
R² étant choisi dans le groupe constitué par halogène, NO₂, CN, C₁-C₆halogénoalkylS(O)ₚ-, C₁-C₆alkyl-S(O)ₚ-, C₂-C₄alcényle-, C₂-C₄alcynyle-, C₁-C₄halogénoalkyle et C₁-C₄halogénoalcoxy- ;
R³ étant choisi dans le groupe constitué par hydrogène, halogène, C₁-C₄alkyle, C₁-C₄halogénoalkyle, C₁-C₄alcoxy- et C₁-C₄halogénoalcoxy- ;
R⁴ étant phényle ou un hétéroaryle à cinq ou six chaînons, l'hétéroaryle contenant de un à trois hétéroatomes chacun indépendamment choisi dans le groupe constitué par oxygène, azote et soufre, et le phényle ou l'hétéroaryle pouvant éventuellement être substitué par un ou plusieurs substituants indépendamment choisis dans le groupe constitué par halogène, C₁-C₆alkyle-, C₂-C₄alcényle-, C₂-C₄alcynyle-, C₁-C₆halogénoalkyle-, C₁-C₆alcoxy-, C₁-C₆alcoxyC₁-C₃alkyle-, C₁-C₆alcoxyC₁-C₃alcoxy-, C₁-C₆halogénoalcoxy-, C₁-C₆alkyl-S(O)ₚ-, C₁-C₆halogénoalkyl-S(O)ₚ-, C₃-C₆cycloalkyle- (éventuellement substitué par 1 ou 2 halogènes), cyano et nitro ; ou
R⁴ étant R4^{a} 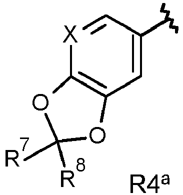 et
R⁵ étant hydrogène ou C₁-C₁₀alkyle ;
R⁶ étant hydrogène ou C₁-C₁₀alkyle ;
R⁷ et R⁸ étant indépendamment choisis dans le groupe constitué par hydrogène et halogène ; et
p = 0, 1 ou 2 ;
ledit composé n'étant pas choisi dans le groupe constitué par 2-(3-bromo-5-cyano-2-hydroxyphényl)pyridine, 3-chloro-2-éthoxy-4-hydroxy-5-phényl-benzonitrile, 4-hydroxy-3-nitro-5-[3-(trifluorométhyl)phényl]benzonitrile et 2'-fluoro-6-hydroxy-5-nitrobiphényl-3-carbonitrile.

2. Composé selon la revendication 1, R¹ étant hydrogène.

3. Composé selon la revendication 1 ou la revendication 2, R² étant choisi dans le groupe constitué par F, Cl, Br, -CF₃ et -OCF₃.

4. Composé selon la revendication 3, R² étant CF₃.

5. Composé selon l'une quelconque des revendications précédentes, R³ étant hydrogène.

6. Composé selon l'une quelconque des revendications 1 à 5, R⁴ étant phényle.

7. Composé selon l'une quelconque des revendications 1 à 5, R⁴ étant un hétéroaryle à cinq ou six chaînons éventuellement substitué choisi dans le groupe constitué par furanyle, thiophényle, thiazolyle, oxazolyle, isoxazolyle, pyrazolyle, isothiazolyle, pyridinyle, pyridazinyle, pyrazinyle, pyrimidinyle et triazolyle.

8. Composé selon la revendication 7, R⁴ étant un hétéroaryle à cinq ou six chaînons éventuellement substitué choisi dans le groupe constitué par pyrazolyle, thiazolyle et thiophényle.

9. Composé selon la revendication 8, R⁴ étant pyridinyle ou pyrazolyle.

10. Composé selon l'une quelconque des revendications précédentes, X étant CH.

11. Composition herbicide comprenant un composé selon l'une quelconque des revendications précédentes et un adjuvant de formulation acceptable sur le plan agricole.

12. Composition herbicide selon la revendication 11, comprenant en outre au moins un pesticide supplémentaire.

13. Composition herbicide selon la revendication 12, le pesticide supplémentaire étant un herbicide ou un phytoprotecteur herbicide.

14. Procédé de lutte contre des mauvaises herbes au niveau d'un site comprenant une application sur le site d'une composition comprenant une quantité de lutte contre des mauvaises herbes d'un composé de formule (I) : 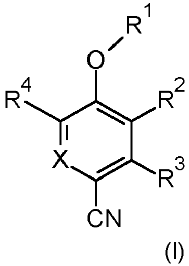 ou d'un sel acceptable sur le plan agronomique correspondant,
X étant CH ou N,
R¹ étant choisi dans le groupe constitué par hydrogène, C₁-C₁₀alkylC(O)-, C₁-C₁₀alkylOC(O)-, C₁-C₁₀alkylSC(O)- et R⁵R⁶NC(O)- ;
R² étant choisi dans le groupe constitué par halogène NO₂, CN, C₁-C₆halogénoalkylS(O)ₚ-, C₁-C₆alkyl-S(O)ₚ-, C₂-C₄alcényle-, C₂-C₄alcynyle-, C₁-C₄halogénoalkyle et C₁-C₄halogénoalcoxy- ;
R³ étant choisi dans le groupe constitué par hydrogène, halogène, C₁-C₄alkyle, C₁-C₄halogénoalkyle, C₁-C₄alcoxy- et C₁-C₄halogénoalcoxy- ;
R⁴ étant phényle ou un hétéroaryle à cinq ou six chaînons, l'hétéroaryle contenant de un à trois hétéroatomes chacun indépendamment choisi dans le groupe constitué par oxygène, azote et soufre, et le phényle ou hétéroaryle pouvant éventuellement être substitué par un ou plusieurs substituants choisis dans le groupe constitué par halogène, C₁-C₆alkyle-, C₂-C₄alcényle-, C₂-C₄alcynyle-, C₁-C₆halogénoalkyle-, C₁-C₆alcoxy-, C₁-C₆alcoxyC₁-C₃alkyle-, C₁-C₆alcoxyC₁-C₃alcoxy-, C₁-C₆halogénoalcoxy-, C₁-C₆alkyl-S(O)ₚ-, C₁-C₆halogénoalkyl-S(O)ₚ-, C₃-C₆cycloalkyle-(éventuellement substitué par 1 ou 2 halogènes), cyano et nitro ; ou
R⁴ étant R4^{a} 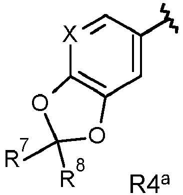 et
R⁵ étant hydrogène ou C₁-C₁₀alkyle ;
R⁶ étant hydrogène ou C₁-C₁₀alkyle ;
R⁷ et R⁸ étant indépendamment choisis dans le groupe constitué par hydrogène et halogène ; et
p = 0, 1 ou 2.

15. Utilisation d'un composé de formule (I) : 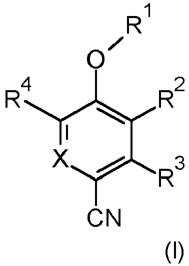 ou d'un sel acceptable sur le plan agronomique correspondant,
X étant CH ou N,
R¹ étant choisi dans le groupe constitué par hydrogène, C₁-C₁₀alkylC(O)-, C₁-C₁₀alkylOC(O)-, C₁-C₁₀alkylSC(O)- et R⁵R⁶NC(O)- ;
R² étant choisi dans le groupe constitué par halogène, NO₂, CN, C₁-C₆halogénoalkylS(O)ₚ-, C₁-C₆alkyl-S(O)ₚ-, C₂-C₄alcényle-, C₂-C₄alcynyle-, C₁-C₄halogénoalkyle et C₁-C₄halogénoalcoxy-;
R³ étant choisi dans le groupe constitué par hydrogène, halogène, C₁-C₄alkyle, C₁-C₄halogénoalkyle, C₁-C₄alcoxy- et C₁-C₄halogénoalcoxy- ;
R⁴ étant phényle ou un hétéroaryle à cinq ou six chaînons, l'hétéroaryle contenant de un à trois hétéroatomes chacun indépendamment choisi dans le groupe constitué par oxygène, azote et soufre, et le phényle ou hétéroaryle pouvant éventuellement être substitué par un ou plusieurs substituants choisis dans le groupe constitué par halogène, C₁-C₆alkyle-, C₂-C₄alcényle-, C₂-C₄alcynyle-, C₁-C₆halogénoalkyle-, C₁-C₆alcoxy-, C₁-C₆alcoxyC₁-C₃alkyle-, C₁-C₆alcoxyC₁-C₃alcoxy-, C₁-C₆halogénoalcoxy-, C₁-C₆alkyl-S(O)ₚ-, C₁-C₆halogénoalkyl-S(O)ₚ-, C₃-C₆cycloalkyle-(éventuellement substitué par 1 ou 2 halogènes), cyano et nitro ; ou
R⁴ étant R4^{a} 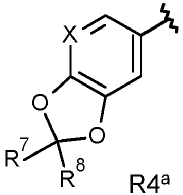 et
R⁵ étant hydrogène ou C₁-C₁₀alkyle ;
R⁶ étant hydrogène ou C₁-C₁₀alkyle ;
R⁷ et R⁸ étant indépendamment choisis dans le groupe constitué par hydrogène et halogène ; et
p = 0, 1 ou 2 ;
en tant qu'herbicide.
